# EUROPÄISCHE PATENTANMELDUNG

(11) **EP 0 163 842 A1**
(43) Veröffentlichungstag der Anmeldung: **11.12.1985**
(21) Anmeldenummer: 85104041.0
(22) Anmeldetag: 03.04.1985
(51) Int. Cl.: C07D 401/12, C07D 491/04, A61K 31/415, A61K 31/44

(54) **Tricyclische Imidazolderivate**

(30) Priorität: 19.04.1984 CH 1966/84
(71) Anmelder: F. HOFFMANN-LA ROCHE AG, 4002 Basel (CH)
(72) Erfinder: Fischli, Albert, Prof, Dr., CH-4125 Riehen (CH); Krasso, Anna, Dr., CH-4054 Basel (CH); Ramuz, Henri, Prof. Dr., CH-4127 Birsfelden (CH); Szente, André, Dr., CH-4125 Riehen (CH)
(74) Vertreter: Lederer, Franz, Dr.

(57) **Zusammenfassung**

Die neuen tricyclischen Imidazolderivate der Formel 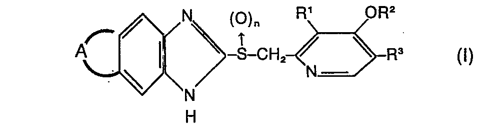 worin eines von R' und R³ niederes Alkyl und das andere Wasserstoff oder niederes Alkyl, R² niederes Alkyl, n die Zahl 0 oder 1, A einen Rest der Formel 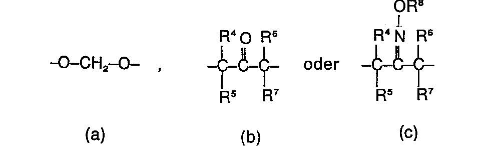
R⁴, R⁵, R⁶ und R⁷ je niederes Alkyl und R⁸ Wasserstoff oder niederes Alkyl bedeuten,

und deren Säureadditionssalze eignen sich zur Bekämpfung bzw. Verhütung von Ulcera und von erhöhter Magensäuresekretion. Sie können nach verschiedenen Verfahren hergestellt und unter Verwendung therapeutisch inerter Excipientien in galenische Darreichungsform gebracht werden.

## Beschreibung

Die vorliegende Erfindung betrifft Imidazolderivate. Im speziellen betrifft sie tricyclische Imidazolderivate der allgemeinen Formel 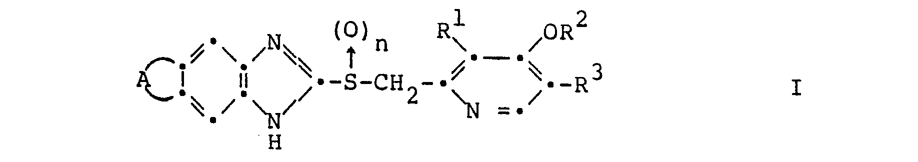 worin eines von R¹ und R³ niederes Alkyl und das andere Wasserstoff oder niederes Alkyl, R² niederes Alkyl, n die Zahl 0 oder 1, A einen Rest der Formel 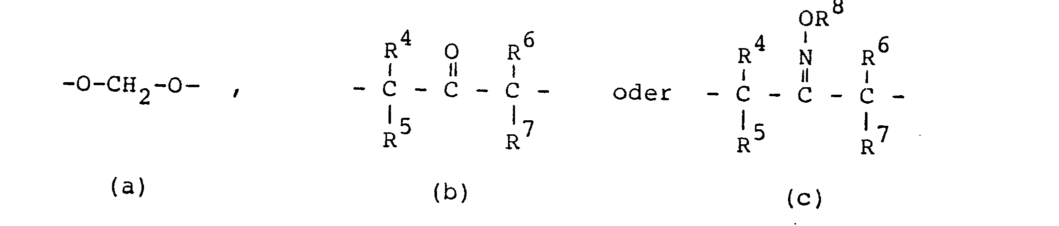 R⁴, R⁵, _{R}⁶ und _{R}⁷ je niederes Alkyl und R⁸ Wasserstoff oder niederes Alkyl bedeuten, und deren Säureadditionssalze.

Diese Verbindungen sind neu und zeichnen sich durch wertvolle pharmakodynamische Eigenschaften aus, indem sie bei geringer Toxizität die Entstehung von Ulcera und die Magensäuresekretion hemmen.

Gegenstand der vorliegenden Erfindungen sind Verbindungen der Formel I und deren Säureadditionssalze als solche und als pharmazeutische Wirkstoffe, die Herstellung und Zwischenprodukte für die Herstellung der Verbindungen der Formel I und ihrer Säureadditionssalze, ferner Arzneimittel, enthaltend eine Verbindung der Formel I oder ein Säureadditionssalz davon und die Herstellung solcher Arzneimittel, sowie die Verwendung der Verbindungen der Formel I und ihrer Säureadditionssalze bei der Bekämpfung bzw. Verhütung von Krankheiten, insbesondere bei der Bekämpfung bzw. Verhütung von Ulcera und von erhöhter Magensäuresekretion, bzw. die Verwendung der Verbindungen der Formel I und ihrer Säureadditionssalze zur Herstellung von Arzneimitteln für die Bekämpfung bzw. Verhütung von Ulcera und von erhöhter Magensäuresekretion.

Im Rahmen der vorliegenden Erfindung bezeichnet der Ausdruck "Alkyl" geradkettige und verzweigte gesättigte Kohlenwasserstoffreste, wie Methyl, Aethyl, n-Propyl, Isopropyl, n-Butyl. Isobutyl und dgl. Der Ausdruck "Alkoxy" bezeichnet Alkyläthergruppen im Sinne der vorstehenden Definition des Ausdrucks "Alkyl". Der Ausdruck "nieder" bezeichnet Reste bzw. Verbindungen mit höchstens 7, vorzugsweise höchstens 4 Kohlenstoffatomen.

Unter den Verbindungen der Formel I sind diejenigen bevorzugt, worin A einen Rest der eingangs definierten Formel (b) oder (c) bedeutet. Dabei haben R⁴, R⁵, _{R}⁶ und R⁷ vorzugsweise dieselbe Bedeutung und bedeuten vorzugsweise je Methyl. Wenn das Symbol A in Formel I einen Rest der eingangs definierten Formel (c) bedeutet, dann bedeutet zudem R⁸ vorzugsweise Wasserstoff oder Methyl.

Bevorzugte Bedeutungsmöglichkeiten für das Symbol R² in Formel I sind Methyl und Aethyl. Was die Symbole R¹ und R³ in Formel I betrifft, so bedeuten vorzugsweise entweder R¹ Wasserstoff und R³ Methyl oder R¹ Methyl und R³ Wasserstoff oder R¹ und R³ je Methyl.

Ein besonders bevorzugtervertreter der von der eingangs definierten Formel I umfassten Klasse tricyclischer Imidazolderivate ist 5,7-Dihydro-2-[[(4-methoxy-3-methyl -2-pyridyl)methyl]-thio] -5,5,7.7-tetramethylindeno[5,6-d]imidazol-6(lH)-on.

Weitere bevorzugte Vertreter dieser Verbindungsklasse sind:
5,7-Dihydro-2-[[(4-methoxy-3-methyl-2-pyridyl)methyl]-sulfinyl]-5,5,7,7-tetramethylindeno[5,6-d]imidazol-6(1H)-on;
2-[[(4-Aethoxy-3-methyl-2-pyridyl)methyl]thio]-5,7-dihydro-5,5,7,7-tetramethylindeno[5,6-d]imidazol-6(lH)-on:
2-[[(4-Aethoxy-3-methyl-2-pyridyl)methyl]sulfinyl]-5,7-dihydro-5,5,7,7-tetramethylindeno[5,6-d]imidazol-6(1H)-on;
5,7-Dihydro-2-[[(4-methoxy-3,5-dimethyl-2-pyridyl)-methyl]thio]-5,5,7,7-tetramethylindeno[5,6-d]imidazol-6(lH)-on;
5,7-Dihydro-2-[[(4-methoxy-3,5-dimethyl-2-pyridyl)-methyl]sulfinyl]-5,5,7,7-tetramethylindeno[5,6-d]imidazol-6(lH)-on;
5,7-Dihydro-2-[[(4-methoxy-5-methyl-2-pyridyl)methyl]-thio]-5,5,7,7-tetramethylindeno[5,6-d]imidazol-6(1H)-on;
5,7-Dihydro-2-[[(4-methoxy-5-methyl-2-pyridyl)methyl]-sulfinyl]-5,5,7,7-tetramethylindeno[5,6-d]imidazol-6(1H)-on;
2-[[(4-Aethoxy-5-methyl-2-pyridyl)methyl]thio]-5,7-dihydro-5,5,7,7-tetramethylindeno[5,6-d]imidazol-6(lH)-on;
2-[[(4-Aethoxy-5-methyl-2-pyridyl)methyl]sulfinyl]-5,7-dihydro-5,5,7,7-tetramethylindeno[5,6-d]imidazol-6(1H)-on:
5,7-Dihydro-2-[[(4-methoxy-3-methyl-2-pyridyl)methyl]-thio]-5.5.7.7-tetrametthylindeno[5,6-d]imidazol-6(1H)-on-O-methyloxim; und
5,7-Dihydro-2-[[(4-methory-3-methyl-2-pyridyl)methyl]-sulfinyl]-5,5,7,7-tetramethylindeno[5,6-d]imidazol-6(1H)-on-O-methyloxim.

Repräsentative Beispiele von Verbindungen der Formel I, worin A einen Rest der eingangs definierten Formel (a) bedeutet, sind:
6-[[(4-Methoxy-3-methyl-2-pyridyl)methyl]thio]-5H-1,3-dioxolo[4,5-f]benzimidazol:
6-[[(4-Methoy-3-methyl-2-pyridyl)methyl]sulfinyl]-5H-1,3-dioxolo[4,5-f]benzimidazol;
6-[[(4-Methoxy-3,5-dimethyl-2-pyridyl)methyl]thio]-5H-1.3-dioxolo[4.5-f]benzimidazol; und
6-[[(4-Methoxy-3,5-dimethyl-2-pyridyl)methyl]sulfinyl]-5H-1,3-dioxolo[4,5-f]benzimidazol.

Die Verbindungen der allgemeinen Formel I und deren Säureadditionssalze können erfindungsgemäss dadurch hergestellt werden, dass man
a) eine Verbindung der allgemeinen Formel 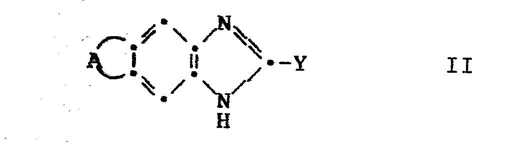 worin A obige und Y die unten angegebene Bedeutung besitzen,
   mit einer Verbindung der allgemeinen Formel 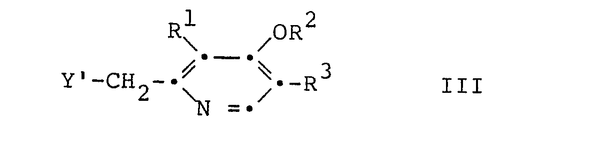 worin R¹, R² und R³ obige und Y¹ die unten angegebene Bedeutung besitzen,
   umsetzt, wobei eines von Y und Y' eine Mercaptogruppe und das andere eine Abgangsgruppe bedeutet; oder
b) eine Verbindung der eingangs definierten Formel I, worin n die Zahl 0 bedeutet, zu einer entsprechenden Verbindung, worin n die Zahl 1 bedeutet, oxydiert; oder
c) eine Verbindung der eingangs definierten Formel I, worin A einen Rest der eingangs definierten Formel (b) und n die Zahl 0 bedeuten, mit einer Verbindung der allgemeinen Formel 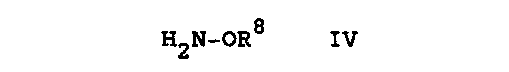 worin R⁸ obige Bedeutung besitzt, umsetzt: oder
d) eine Verbindung der allgemeinen Formel 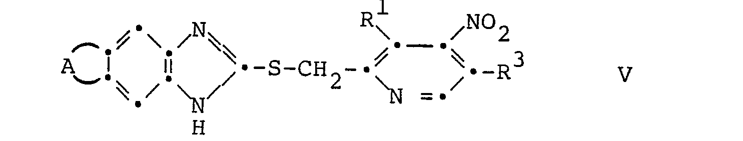 worin R^{1.} R³ und A obige Bedeutung besitzen.
   mit einem eine niedere Alkoxygruppe liefernden Mittel behandelt; oder
e) eine Verbindung der allgemeinen Formel 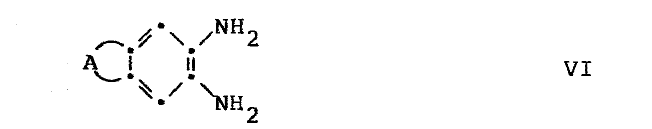 worin A obige Bedeutung besitzt, mit einer Verbindung der allgemeinen Formel 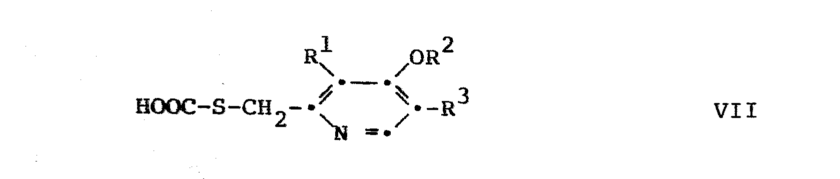 worin R¹, R² und R³ obige Bedeutung besitzen, umsetzt; oder
f) eine Verbindung der allgemeinen Formel 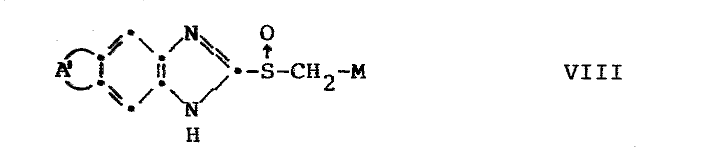 worin A' einen Rest der eingangs definierten Formel (a), (b) oder (c) bedeutet, wobei aber in Formel (c) R⁸ niederes Alkyl bedeutet, und M ein Alkalimetallatom bedeutet,
   mit einer Verbindung der allgemeinen Formel 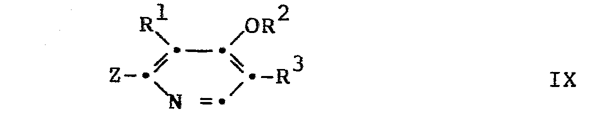 worin R¹, R² und R³ obige Bedeutung besitzen und Z eine Abgangsgruppe bedeutet, umsetzt: und erwünschtenfalls eine erhaltene freie Base in ein Säureadditionssalz und/oder ein erhaltenes Säureadditionssalz in die freie Base oder in ein anderes Säureadditionssalz überführt.

In einer besonderen Ausführungsform des erfindungsgemässen Verfahrens werden Verbindungen der Formel I, worin A einen Rest der eingangs definierten Formel (a) bedeutet, gemäss Verfahrensvariante d), e) oder f) hergestellt.

Nach einem ersten erfindungsgemässen Verfahrensaspekt wird somit eine Verbindung der allgemeinen Formel II mit einer Verbindung der allgemeinen Formel III umgesetzt, wobei entweder das Symbol Y in Formel II eine Mercaptogruppe und das Symbol Y' in Formel III eine Abgangsgruppe oder aber das Symbol Y in Formel II eine Abgangsgruppe und das Symbol Y' in Formel III eine Mercaptogruppe bedeuten. Abgangsgruppen sind beispielsweise Halogenatome, insbesondere Chlor, Brom oder Jod, oder geeignete Säurereste, insbesondere Reste starker organischer Sulfonsäuren, z.B. Arylsulfonyloxyreste, wie Tosyloxy, oder Alkylsulfonyloxyreste, wie Mesyloxy. Weitere Beispiele von Abgangsgruppen sind Alkylsulfinylreste, wie Methylsulfinyl. Die Umsetzung der Verbindungen der Formeln II und III erfolgt zweckmässigerweise in Gegenwart eines unter den Reaktionsbedingungen inerten Lösungsmittels oder Lösungsmittelgemisches und gegebenenfalls in Gegenwart einer Base. Als Basen eignen sich hierfür insbesondere anorganische Basen, wie Natrium- oder Kaliumhydroxyd, Natrium- oder Kaliumcarbonat und dgl., oder organische Basen, wie Triäthylamin oder andere tertiäre Amine. Als Lösungsmittel bzw. Lösungsmittelgemische eignen sich in erster Linie Alkohole, wie Aethanol, Gemische von Alkoholen und Wasser, Aceton, Aether, wie Tetrahydrofuran, halogenierte Kohlenwasserstoffe, wie Methylenchlorid oder Chloroform, Dimethylformamid usw. Die Reaktionstemperatur ist in ziemlich weiten Grenzen variierbar, und sie liegt zweckmässigerweise zwischen etwa Raumtemperatur und etwa der Siedetemperatur des Reaktionsgemisches.

Nach einem zweiten erfindungsgemässen Verfahrensaspekt wird eine Verbindung der Formel I, worin n die Zahl 0 bedeutet, zu einer entsprechenden Verbindung, worin n die Zahl 1 bedeutet, oxydiert. Dabei wird also ein Schwefelatom in die Sulfinylgruppe übergeführt, und man verwendet demgemäss hierfür Oxydationsmittel, welche für derartige Ueberführungen gebräuchlich sind, beispielsweise Persäuren, wie m-Chlorperbenzoesäure, Wasserstoffperoxyd, Perester, Natriummetaperjodat, usw. Die Oxydation erfolgt zweckmässigerweise in einen unter den Reaktionsbedingungen inerten organischen Lösungsmittel, beispielsweise in einen halogenierten Kohlenwasserstoff, wie Methylenchlorid, Chloroform, Dichloräthan und dgl. oder in einen Kohlenwasserstoff, wie Benzol und dgl.: bei Verwendung von Wasserstoffperoxyd als Oxydationsmittel kann auch in Essigsäure, wässriger Essigsäure und dgl. gearbeitet werden. Es ist von Vorteil. das Oxydationsmittel in leichten Ueberschuss bezüglich des zu oxydierenden Produktes einzusetzen. Zweckmässigerweise wird bei Raumtemperatur oder darunter gearbeitet, vorzugsweise bei Temperaturen von etwa -50 bis etwa 0°C.

Nach einem weiteren erfindungsgemässen Verfahrensaspekt werden Verbindungen der Formel I, worin A einen Rest der Formel (b) und n die Zahl 0 bedeuten, mit einer Verbindung der allgemeinen Formel IV umgesetzt. Die Verbindung der Formel IV wird zweckmäseigerweise in Form eines Säureadditionssalzes eingesetzt, beispielsweise als Hydrochlorid. Die Reaktion erfolgt zweckmässigerweise in einem unter den Reaktionsbedingungen inerten Lösungsmittel, beispielsweise in einem Alkohol, wie Methanol, Aethanol und dgl. oder in Mischungen davon mit Wasser. Es kann von Vorteil sein, in Gegenwart eines säurebindenden Mittels zu arbeiten, wie z.B. Kalium- oder Natriumcarbonat und dgl. Die Reaktionstemperatur ist nicht kritisch: man kann zweckmässigerweise zwischen etwa Raumtemperatur und Siedetemperatur des Reaktionsgemisches arbeiten.

Nach einem weiteren erfindungsgemässen Verfahrensaspekt wird eine Verbindung der Formel V mit einem eine niedere Alkoxygruppe liefernden Mittel behandelt. Zweckmässigerweise geht man so vor, dass man die Verbindung der Formel V während längerer Zeit, z.B. während etwa 4-24 Stunden, in einer Lösung eines niederen Alkalimetall-alkoxyds im entsprechenden niederen Alkanol, beispielsweise in methanolischem Natriummethylat oder in äthanolischem Natriumäthylat, erhitzt. Der Zusatz einer anorganischen Base, wie Natriumcarbonat und dgl., kann von Vorteil sein. Zweckmässigerweise arbeitet man bei einer Temperatur zwischen etwa 40°C und der Siedetemperatur des Reaktionsgemisches.

Nach einem weiteren erfindungsgemässen Verfahrensaspekt wird eine Verbindung der Formel VI mit einer Verbindung der Formel VII umgesetzt. Diese Reaktion erfolgt zweckmässigerweise in einem polaren..gegebenenfalls wasserhaltigen Lösungsmittel in Gegenwart einer starken Säure, wie Salzsäure und dgl. Weiterhin erfolgt die Reaktion zweckmässigerweise etwa bei der Siedetemperatur des verwendeten Lösungsmittels oder Lösungsmittelgemisches.

Nach einem weiteren erfindungsgemässen Verfahrensaspekt wird eine Verbindung der Formel VIII mit einer Verbindung der Formel IX umgesetzt. Das in Formel VIII durch das Symbol M wiedergegebene Alkalimetallatom ist zweckmässigerweise ein Lithium-, Natrium- oder Kaliumatom. Als Abgangsgruppe (Z in Formel IX) eignen sich beispielsweise reaktionsfähige Halogenatome, insbesondere Chloratome, oder durch Veresterung aktivierte Hydroxylgruppen, z.B. Arylsulfonyloxyreste, wie Tosyloxy, oder Alkylsulfonyloxyreste, wie Mesyloxy. Die Reaktion erfolgt zweckmässigerweise in einem inerten Lösungsmittel, beispielsweise in einem aromatischen Kohlenwasserstoff, wie Benzol oder Toluol. Die Reaktionstemperatur liegt in der Regel zwischen etwa 0°C und etwa 120°C, wobei die Siedetemperatur des verwendeten Lösungsmittels bevorzugt ist.

Je nach der Verfahrensbedingung und den eingesetzten Ausgangsmaterialien erhält man die Verbindungen der Formel I entweder als freie Basen oder als Säureadditionssalze. Die freien Basen können durch Umsetzung mit organischen oder anorganischen Säuren in entsprechende Säureadditionssalze übergeführt werden, wobei die Verwendung solcher Säuren bevorzugt ist, welche therapeutisch verträgliche Salze bilden, beispielsweise Chlorwasserstoff, Bromwasserstoff, Phosphorsäure, Schwefelsäure, Zitronensäure, p-Toluolsulfonsäure und dgl. Die Säureadditionssalze der Verbindungen der Formel I können in an sich bekannter Weise in die entsprechenden freien Basen oder in andere Säureadditionssalze übergeführt werden. Die Säureadditionssalze von Verbindungen der Formel I, worin n die Zahl 1 bedeutet, sind in wässriger Lösung wenig stabil.

Die Ausgangsprodukte der allgemeinen Formel V sind neu und ebenfalls Gegenstand der vorliegenden Erfindung. Ihre Herstellung kann in Analogie zu dem erfindungsgemässen Verfahrensaspekt a) erfolgen, indem man eine Verbindung der Formel II mit einer Verbindung der allgemeinen Formel 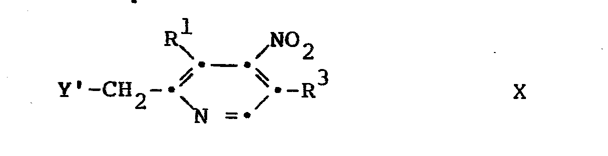 worin R¹, R³ und Y' obige Bedeutung besitzen, umsetzt.

Die Ausgangsprodukte der Formeln II-IV und VI-X sind bekannt oder nach an sich bekannten und jedem Fachmann geläufigen Methoden leicht herstellbar; zudem enthalten einige der nachfolgenden Beispiele detaillierte Angaben betreffend die Herstellung bestimmter Verbindungen der Formeln II, III und X, worin Y bzw. Y' Chlor bedeutet, und der Formel III, worin Y' eine Mercaptogruppe bedeutet.

Wie eingangs erwähnt, sind die tricyclischen Imidazolderivate der Formel I und deren Säureadditionssalze neue Verbindungen mit wertvollen pharmakodynamischen Eigenschaften.

Repräsentative Verbindungen der Formel I wurden auf ihre Antiulcuswirkung, auf ihre magensäuresekretionshemmende Wirkung sowie auf ihre Toxizität untersucht.

Zur Bestimmung der Antiulcuswirkung wurde die nachstehend beschriebene Versuchsanordnung verwendet:
Für jede Dosis einer Testsubstanz verwendet man Gruppen von je 8 männlichen Ratten mit einem Körpergewicht von 130-150 g. Vor Beginn des Versuchs erhalten die Tiere während 24 Stunden keine Nahrung, jedoch Wasser ad libitum. Verschiedene Dosen der zu prüfenden Substanzen (suspendiert in 0,5% Tragacanth) oder das Vehikel allein (Kontrolle) werden zweimal peroral verabreicht, und zwar 1 Stunde bevor und 2 Stunden nach peroraler Verabreichung von 20 mg/kg Indomethacin. Bei Kontrolltieren führt diese Dosis von Indomethacin innerhalb von 5 Stunden zu Läsionen des Magens. 6 Stunden nach der ersten Verabreichung der zu untersuchenden Substanz (bzw. des Vehikels allein) werden die Tiere getötet. Die Ratten, welche vor dem Auftreten makroskopisch sichtbarer Läsionen der Magenschleimhaut geschützt geblieben sind, werden gezählt. Als ED₅₀ bezeichnet man diejenige Dosis einer Testsubstanz, bei welcher 50% der Tiere vor dem Auftreten solcher Läsionen geschützt sind.

Zur Bestimmung der magensäuresekretionhemmenden Wirkung wurde die nachstehend beschriebene Versuchsanordnung verwendet:
Weiblichen und männlichen Beagle-Hunden wird ein Teil des Magenfundus vom restlichen Magen in Form einer Tasche vom Heidenhain-Typ abgetrennt (Modifikation der von Rudick et, al. in J, Surgical Research 7. 383-398 (1967) beschriebenen Methode). In die Tasche wird eine Stahlkanüle eingenäht, welche durch die Bauchdecke nach aussen geleitet wird. Vor jedem Versuch erhalten die Tiere während 18 Stunden keine Nahrnng, jedoch Wasser ad libitum. Während des Versuchs sind sie wach und stehen, und ihre Magensäuresekretion wird durch intravenöse Infusion von 4-Methylhistaain, einen selektiven Agonisten der Histamin-H₂--Rezeptoren. stimuliert. Die Magensäureproduktion wird in 15-Minuten-Fraktionen des Magentaschensaftes bestimmt. Sobald die Magensäureproduktion einen konstanten Wert aufweist, werden die zu prüfenden Substanzen, als trockene Pulver in Gelatinekapseln gefüllt, oral verabreicht. Als ED₅₀ bezeichnet man diejenige Dosis einer Testsubstanz, welche bei den behandelten Tieren im Vergleich zu Kontrolltieren eine 50%ige Hemmung der durch 4-Methylhistamin hervorgerufenen Magensäureproduktion bewirkt.

In der nachfolgenden Tabelle werden für eine Reihe repräsentativer Verbindungen der Formel I die Resultate der Prüfung auf ihre Antiulcuswirkung und auf ihre magensekretionshemmende Wirkung wiedergegeben. Ausserdem enthält diese Tabelle Angaben über die akute Toxizität (DL₅₀ bei einmaliger oraler Verabreichung an Mäuse).

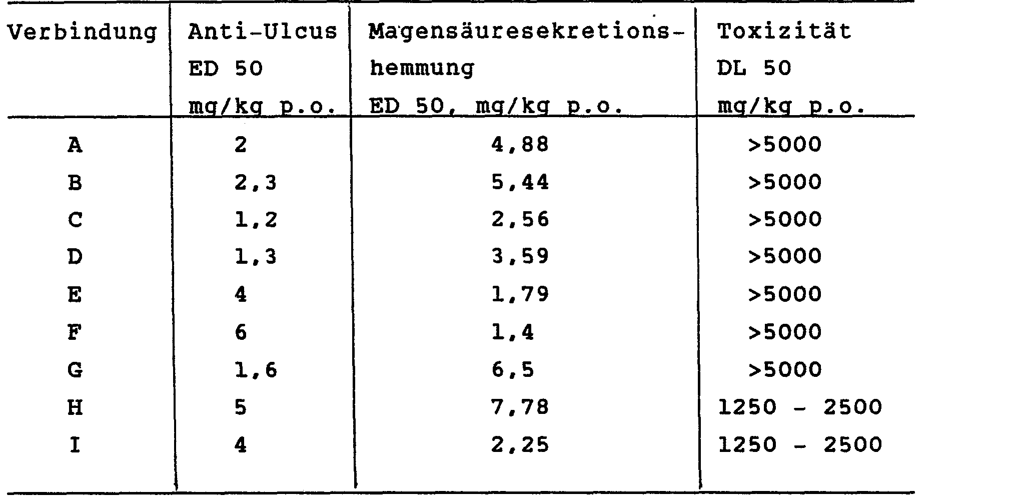

A: 5.7-Dihydro-2-[[(4-methoxy-3,5-dimethyl-2-pyridyl)methyl] thio]-5,5,7,7-tetramethylindeno[5,6-d]imidazol-6(1H)-on
B: 5,7-Dihydro-2-[[(4-methoxy-3,5-dimethyl-2-pyridyl)methyl] sulfinyl]-5,5,7,7-tetramethylindeno[5,6-d]imidazol-6(1H)-on
C: 5,7-Dihydro-2-[[(4-methoxy-3-methyl-2-pyridyl)methyl] thio]-5.5.7,7-tetramethylindeno[5,6-d]imidazol-6(lH)-on
D: 5,7-Dihydro-2-[[(4-methoxy-3-methyl-2-pyridyl)methyl]sulfinyl]-5,5,7,7-tetramethylindeno[5,6-d]imidazol-6(lH)-on
E: 6-[[(4-Methoxy-3-methyl-2-pyridyl)methyl]sulfinyl]-5H-1,3-dioxolo[4,5-f]benzimidazol
F: 6-[[(4-Methoxy-3,5-dimethyl-2-pyridyl)methyl]sulfinyl]-5H-1,3-dioxolo[4,5-f]benzimidazol
G: 2-[[(4-Methoxy-3-methyl-2-pyridyl)methyl]thio]-5,7-dihydro-5,5,7,7-tetramethylindeno[5,6-d]imidazol-6(1H)-on
H: 5,7-Dihydro-2-[[(4-methoxy-3-methyl-2-pyridyl)methyl]-sulfinyl]-5,5,7,7-tetramethylindeno[5,6-d]imidazol-6(lH)-on-0-methyloxim
I: 5,7-Dihydro-2-[[(4-methoxy-3-methyl-2-pyridyl)methyl]-thio]-5,5,7,7-tetramethylindeno[5,6-d]imidazol-6(1H)-on-0-methyloxim.

Die Verbindungen der Formel I und Säureadditionssalze davon können als Heilmittel, z.B. in Form pharmazeutischer Präparate Verwendung finden. In erster Linie kommt orale Verabreichung in Form von festen pharmazeutischen Präparaten, wie Tabletten, Lacktabletten, Dragees, Hartgelatinekapseln und Weichgelatinekapseln in Frage. Falls in Formel I n die Zahl 1 bedeutet, müssen diese pharmazeutischen Präparate magensaftresistent sein. Orale Verabreichung in Form flüssiger pharmazeutischer Präparate, wie Lösungen, Emulsionen und Suspensionen, rektale Verabreichung. z.B. in Form von Suppositorien, oder parenterale Verabreichung, z.B. in Form von Injektionslösungen, sind zwar weniger in Betracht zu ziehen aber auch nicht auszuschliessen.

Wie eingangs erwähnt, sind Arzneimittel, enthaltend eine Verbindung der Formel I oder ein Säureadditionssalz davon, ebenfalls Gegenstand der vorliegenden Erfindung, weiterhin auch ein Verfahren zur Herstellung derartiger Arzneimittel, welches dadurch gekennzeichnet ist, dass man eine oder mehrere Verbindungen der Formel I oder Säureadditionssalze davon und erwünschtenfalls einen oder mehrere andere therapeutische Wirkstoffe zusammen mit einem oder mehreren therapeutisch inerten Excipientien in eine galenische Darreichungsform bringt.

Zur Herstellung von Tabletten, Lacktabletten, Dragees und Hartgelatinekapseln können die Verbindungen der Formel I oder Säureadditionssalze davon mit pharmazeutisch inerten, anorganischen oder organischen Excipientien verarbeitet werden. Als solche Excipientien kann man z.B. für Tabletten, Dragees und Hartgelatinekapseln Lactose, Maisstärke oder Derivate davon, Talk, Stearinsäure oder deren Salze etc. verwenden. Zur Herstellung von magensaftresistenten pharmazeutischen Präparaten ist noch ein magensaftresistenter Lack aufzubringen, welcher z.B. aus Hydroxypropylmethylcellulosephthalat bestehen kann.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glucose und dgl.

Für Suppositorien eignen sich als Excipientien z.B. natürliche oder gehärtete Oele, Wachse, Fette, halbflüssige oder flüssige Polyole und dgl.

Für Injektionslösungen eignen sich als Excipientien z.B. Wasser, Alkohole, Polyole, Glycerin, vegetabile Oele etc.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der Formel I und Säureadditionssalze davon bei der Bekämpfung bzw. Verhütung von Krankheiten verwenden, beispielsweise bei der Bekämpfung bzw. Verhütung von Ulcera und von erhöhter Magensäuresekretion. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 30-400 mg und bei intravenöser Verabreichung eine Tagesdosis von etwa 1-50 mg angemessen sein.

Gegenstand der Erfindung ist, wie eingangs erwähnt, auch die Verwendung der Verbindungen der Formel I und ihrer Säureadditionssalze zur Herstellung von Arzneimitteln für die Bekämpfung bzw. Verhütung von Ulcera und von erhöhter Magensäuresekretion.

In den nachfolgenden Beispielen, welche die vorliegende Erfindung illustrieren, ihren Umfang aber in keiner Weise beschränken sollen, sind sämtliche Temperaturen in Celsiusgraden angegeben.

### Beispiel 1

a) Eine Lösung von 24 g (0,22 Mol) 2,3-Dimethylpyridin in 100 ml Methylenchlorid wird unter Eiskühlung mit einer Lösung von 46.6 g (0,27 Mol) m-Chlorperbenzoesäure in 100 ml Methylenchlorid versetzt. Man erhitzt das Reaktionsgemisch 2 Stunden unter Rückfluss und engt im Rotationsverdampfer ein. Der Rückstand wird an Silicagel mit Essigester/Methylenchlorid (3:1) als Elutionsmittel chromatographiert, wobei die Methode der Mitteldruck-Flashchromatographie verwendet und der Druck mit Stickstoffgas erzeugt wird. Durch Umkristallisation aus Aether erhält man 2,3-Dimethylpyridin-1-oxid von Schmelzpunkt 56°.
b) Eine Lösung von 15 g (0,12 Mol) 2,3-Dimethylpyridin-1-- oxid in 75 ml Chloroform wird am Rückfluss gekocht und so schnell wie möglich mit 37 ml Trichloressigsäurechlorid versetzt (es ist vorteilhaft, das Säurechlorid durch den Rückflusskühler zuzugeben). Man erhitzt das Reaktionsgemisch 2,5 Stunden unter Rückfluss, giesst es anschliessend auf ein Gemisch von Eis und Natriumbicarbonat und wäscht die entstandene Lösung mehrmals mit Methylenchlorid. Die organische Phase wird mit Natriumsulfat getrocknet, filtriert und eingeengt. Der Rückstand wird an Silicagel mit Methylenchlorid chromatographiert, wobei die Methode der Mitteldruck-Flashchromatographie verwendet und der Druck mit Stickstoffgas erzeugt wird. Das erhaltene 2-Chlormethyl-3-methylpyridin wird direkt weiterverarbeitet.
c) Eine Lösung von 24 g (0,17 Mol) 2-Chlormethyl-3-methyl- pyridin in 200 ml Methylenchlorid wird unter Eiskühlung mit einer Lösung von 44 g (0,25 Mol) m-Chlorperbenzoesäure in 200 ml Methylenchlorid versetzt. Man erhitzt das Reaktionsgemisch 2 Stunden unter Rückfluss und engt im Rotationsverdampfer ein. Der Rückstand wird an Silicagel mit Essigester/Methylenchlorid (3:1) als Elutionsmittel chromatographiert, wobei die Methode der Mitteldruck-Flashchromatographie verwendet und der Druck mit Stickstoffgas erzeugt wird. Das erhaltene 2-Chlormethyl-3-methyl-pyridin-l-oxid wird direkt weiterverarbeitet.
d) Zu 230 ml konzentrierter Salpetersäure (68%ig: d = 1,41) gibt man unter Trockeneiskühlung langsam 300 ml konzentrierte Schwefelsäure zu, wobei die Temperatur des Gemisches 5° nicht übersteigt. Man gibt eine Lösung von 38,7 g (0,25 Mol) 2-Chlormethyl-3-methylpyridin-l-oxid zu und rührt während 2 Stunden bei 80°. Das Reaktionsgemisch wird auf ein Gemisch von Eis und Methylenchlorid gegossen, die wässerige Phase wird mehrmals mit Methylenchlorid gewaschen, und die Methylenchloridlösung wird mit 10%iger Natriumbicarbonatlösung extrahiert. Die organische Phase wird mit Natriumsulfat getrocknet und eingeengt. Der Rückstand wird aus Essigester umkristallisiert, wobei man 2-Chlormethyl-3-methyl-4-nitropyridin-l-oxid erhält. Das Produkt zeigt einen Schmelzpunkt von 126-129°.
e) Eine Lösung von 4,5 g (0,024 Mol) 2-Chlormethyl-3-- methyl-4-nitropyridin-l-oxid in 25 ml Methylenchlorid und 25 ml Acetonitril wird mit 5 ml Phosphortrichlorid versetzt und 20 Minuten bei Raumtemperatur gerührt. Das Reaktionsgemisch wird auf ein Gemisch von Eis und 20 g Natriumcarbonat gegossen, und die entstandene wässerige Lösung wird mehrmals mit Methylenchlorid gewaschen. Die organische Phase wird getrocknet und eingedampft. Das so erhaltene 2-Chlormethyl-3-methyl-4-nitropyridin wird direkt weiterverarbeitet.
f) Eine Lösung von 11.5 g (0,062 Mol) 2-Chlormethyl-3-- methyl-4-nitropyridin und 16 g (0,06 Mol) 5,7-Dihydro-2-- mercapto-5,5,7,7-tetramethylindeno[5,6-d]imidazol-6(1H)-on in 200 ml abs. Aceton wird mit 13 g fein gemahlenem Kaliumcarbonat versetzt und bei Raumtemperatur unter Argon 18 Stunden gerührt. Im Vakuum werden 100 ml Aceton abdestilliert, worauf der Rest auf Eis gegossen wird. Das auskristallisierte Produkt wird abfiltriert und in Methylenchlorid gelöst: die erhaltene Lösung wird mit Wasser gewaschen, getrocknet und eingeengt. Durch Umkristallisation aus Essigester/Aether erhält man 5,7-Dihydro-5,5,7,7-tetramethyl-2-[[(3-methyl-4-nitro-2-pyridyl)methyl]thio]indeno-[5,6-d]imidazol-6(1H)-on vom Schmelzpunkt 181-183° (Zersetzung).
g) Eine Lösung von 4,4 g (0,011 Mol) 5,7-Dihydro-5,5,7,7-- tetramethyl-2-[[(3-methyl-4-nitro-2-pyridyl)methyl]thio]-indeno[5,6-d]imidazol]-6(1H)-on in 100 ml abs. Methanol wird mit 3 g Natriummethylat versetzt, worauf während 18 Stunden unter Argon am Rückfluss gekocht wird. Nach Einengen des Reaktionsgemisches im Vakuum wird der Rückstand mit Methylenchlorid versetzt, worauf man mittels Eisessig puffert: die Methylenchloridphase wird mehrmals mit einer Natriumbicarbonatlösung extrahiert, getrocknet und eingedampft. Durch Umkristallisation aus Essigester erhält man 5,7-Dihydro-2-[[(4-methoxy-3-methyl-2-pyridyl)methyl]thio]--5,5,7,7-tetramethylindeno[5,6-d]imidazol-6(1H)-on vom Schmelzpunkt 222-226°.

### Beispiel 2

Eine Lösung von 6 g (0,015 Mol) 5,7-Dihydro-2-[[(4-- methoxy-3-methyl-2-pyridyl)methyl]thio]-5,5,7,7-tetramethyl- indeno[5,6-d]imidazol-6(lH)-on in 90 ml abs. Methylenchlorid wird unter Argon bei -40 bis -50° innerhalb von 10 Minuten mit einer Lösung von 3,3 g (0,019 Mol) m-Chlorperbenzoesäure in 50 ml abs. Methylenchlorid versetzt. Anschliessend wird die Lösung noch zusätzlich 20 Minuten gerührt, mit einer 10%igen Natriumcarbonatlösung extrahiert, getrocknet und unter stetigem Ersatz von Methylenchlorid durch Essigester eingedampft. Dabei kristallisiert 5,7-Di- hydro-2-[[(4-methoxy-3-methyl-2-pyridyl)methyl]sulfinyl]-5,5, 7,7-tetramethylindeno[5,6-d]imidazol-6(lH)-on, welches bei 192-194° unter Zersetzung schmilzt.

### Beispiel 3

Ein Gemisch von 10,5 g Natriumäthylat, 20 g Natriumcarbonat und 400 ml Aethanol wird unter Argon bei 50° während 30 Minuten gerührt und dann mit 7 g (0,017 Mol) 5,7-Dihy- dro-5,5.7,7-tetramethyl-2-[[(3-methyl-4-nitro-2-pyridyl)-methyl]thio]indeno[5,6-d]imidazol-6(1H)-on versetzt. Nach 5-stündigem Rühren bei 50° unter Argon wird das Reaktionsgemisch im Vakuum eingeengt. Der Rückstand wird mit Methylenchlorid versetzt und mittels Eisessig gepuffert; die Methylenchloridlösung wird mit Natriumbicarbonatlösung extrahiert, getrocknet und eingeengt. Der Rückstand wird an Kieselgel unter Eluieren mit Methylenchlorid/Essigester (3:1) chromatograhiert, wobei die Methode der Mitteldruck-Flashchromatographie verwendet und der Druck mit Stickstoffgas erzeugt wird. Man erhält 2-[[(4-Aethoxy-3-methyl--2-pyridyl)methyl]thio]-5,7-dihydro-5,5,7,7-tetramethylindeno-[5,6-d]imidazol-6(lH)-on, welches nach Umkristallisieren aus Aether bei 175-176° schmilzt.

### Beispiel 4

Eine Lösung von 2 g (0,0049 Mol) 2-[[(4-Aethoxy-3-- methyl-2-pyridyl)methyl]thio]-5,7-dihydro-5,5,7,7-tetramethyl indeno[5,6-d]imidazol-6(1H)-on in 35 ml abs. Methylenchlorid wird unter Argon bei -40 bis -50° innerhalb 10 Minuten mit 1,1 g (0,0064 Mol) m-Chlorperbenzoesäure in 10 al abs. Methylenchlorid versetzt. Anschliessend wird die Lösung noch zusätzlich 20 Minuten gerührt, mit 10%iger Natriumcarbonatlösung extrahiert, getrocknet und unter stetigen Ersatz von Methylenchlorid durch Essigester eingeengt. Dabei kristallisiert 2-[[(4-Aethoxy-3-methyl-2-pyridyl)methyl]-sulfinyl]-5,7-dihydro-5,5,7,7-tetramethylindeno[5,6-d]imidazol-6(1H)-on, welches bei 180° unter Zersetzung schmilzt.

### Beispiel 5

Eine Lösung von 560 mg Kaliumhydroxid in 1,4 al Methanol und eine Lösung von 700 mg Hydroxylamin-hydrochlorid in 3,6 ml Methanol werden bei 40° miteinander vermischt, worauf man 30 Minuten rührt und 300 mg 5,7-Dihydro-2[[(4-- methoxy-3-methyl-2-pyridyl)methyl]thio]-5,5,7,7-tetramethyl- indeno[5.6-d]imidazol-6(1H)-on zugibt. Das Reaktionsgemisch wird bei 40° unter Argon während 3 Tagen gerührt und dann eingeengt und mit Methylenchlorid/Wasser versetzt. Die Methylenchloridlösung wird abgetrennt, getrocknet, filtriert und eingedampft. Der Rückstand wird zweimal an Silicagel mit Methylenchlorid/Methanol (9:1) chromatographiert, wobei die Methode der Mitteldruck-Flashchromatographie verwendet und der Druck mit Stickstoffgas erzeugt wird. Man erhält 5,7-Dihydro-2-[[(4-methoxy-3-methyl-2-pyridyl)-methyl]thio]-5,5,7,7-tetramethylindeno[5,6-d]imidazol-6(1H)-on-oxim. Nach Umkristallisieren aus Essigester schmilzt das Produkt bei 160° unter Zersetzung.

### Beispiel 6

Eine Lösung von 41 mg (0,01 mMol) 5,7-Dihydro-2-[[(4-- methoxy-3-methyl-2-pyridyl)methyl]thio]-5,5,7,7-tetramethyl- indeno[5,6-d]imidazol-6(lH)-on-oxim in 2 ml abs. Methylenchlorid wird unter Argon bei -40 bis -50° innerhalb von 10 Minuten mit 22,5 mg (0,13 mMol) m-Chlorperbenzoesäure in 1 ml abs. Methylenchlorid versetzt. Die Lösung wird noch zusätzlich während 15 Minuten gerührt und dann mit 10%iger Natriumcarbonatlösung extrahiert, getrocknet und unter stetigem Ersatz von Methylenchlorid durch Essigester eingeengt. Dabei kristallisiert 5,7-Dihydro-2-[[(4-methoxy-3-- methyl-2-pyridyl)methyl]sulfinyl]-5,5,7,7-tetramethylindeno-[5,6-d]imidazol-6(lH)-on-oxim, welches bei 215-217⁰ schmilzt.

### Beispiel 7

Eine Lösung von 6,5 g (0.016 Mol) 5,7-Dihydro-2-[[(4-- methoxy-3-methyl-2-pyridyl)methyl]thio]-5.5,7,7-tetramethyl- indeno[5,6-d]imidazol-6(1H)-on in 50 ml abs. Methanol wird mit 6 g O-Methylhydroxylamin-hydrochlorid versetzt. Das Reaktionsgemisch wird 60 Stunden unter Argon am Rückfluss gekocht und dann eingeengt und mit Methylenchlorid/Natriumcarbonatlösung extrahiert. Die Methylenchloridlösung wird abgetrennt, filtriert, getrocknet und eingedampft. Der Rückstand wird an Silicagel mit einer Lösung von wasserfreiem Ammoniak in Aether chromatographiert, wobei die Methode der Mitteldruck-Flashchromatographie verwendet und der Druck mit Stickstoffgas erzeugt wird. Man erhält 5,7-Dihydro-3-[[(4-methoxy-3-methyl-2-pyridyl)methyl]thio]--5,5,7,7-tetramethylindeno[5,6-d]imidazol-6(lH)-on-O-methyloxim, welches nach Umkristallisieren aus n-Hexan/Aether einen Schmelzpunkt von 100° aufweist.

### Beispiel 8

Eine Lösung von 540 mg (1,27 mMol) 5,7-Dihydro-3-[[(4-- methoxy-3-methyl-2-pyridyl)methyl]thio]-5,5,7,7-tetramethyl- indeno[5,6-d]imidazol-6(1H)-on-O-methyloxim in 15 ml abs. Methylenchlorid wird unter Argon bei -40 bis -50° innerhalb von 10 Minuten mit 290 mg (1,7 mMol) m-Chlorperbenzoesäure in 5 ml abs. Methylenchlorid versetzt. Die Lösung wird noch zusätzlich während 20 Minuten gerührt und dann mit 10%iger Natriumcarbonatlösung extrahiert, getrocknet und eingeengt. Der aus Aether kristallisierte Rückstand ergibt 5,7-Dihy- dro-2-[[(4-methoxy-3-methyl-2-pyridyl)methyl]sulfinyl]-5,5,-7,7-tetramethylindeno[5,6-d]imidazol-6(1H)-on-O-methyloxim von Schmelzpunkt 140° (Zersetzung).

### Beispiel 9

a) Eine Lösung von 183 mg (1.49 mMol) 2,3-Dimethylpyridin-1-oxid in 0,6 ml konz. Schwefelsäure wird unter Eiskühlung mit 0,2 ml 65%iger Salpetersäure (d=1.4) versetzt. Das Reaktionsgemisch wird 24 Stunden bei 90° gerührt und auf ein Gemisch von Eis und Natriumcarbonat gegossen, worauf man mit Methylenchlorid extrahiert und die Methylenchloridphase trocknet und eindampft. Der aus Aethanol/n-Pentan kristallisierte Rückstand ergibt 2,3-Dimethyl-4-- nitropyridin-1-oxid vom Schmelzpunkt 99-102°.
b) Eine Lösung von 2,5 g (0,015 Mol) 2.3-Dimethyl-4-nitro- pyridin-1-oxid in 50 ml abs. Methanol wird mit 0.883 g Natriummethylat versetzt, worauf unter Argon bei Raumtemperatur während 2 Tagen gerührt wird. Die Reaktionsmischung wird eingeengt, und der Rückstand wird mit Methylenchlorid und gesättigter Natriumchloridlösung extrahiert. Die Methylenchloridphase wird getrocknet und eingedampft. Der aus Methylenchlorid/Aether kristallisierte Rückstand ergibt 4-Methoxy-2,3-dimethylpyridin-1-oxid vom Schmelzpunkt 80-83°.
c) Eine Lösung von 500 mg (3,26 mMol) 4-Methoxy-2,3-- dimethylpyridin-l-oxid in 20 ml 1,2-Dichloräthan wird am Rückfluss gekocht und mit 8,3 g Trichloressigsäurechlorid versetzt. Nach 35 Minuten wird das Reaktionsgemisch auf Eis gegossen, worauf man 10%ige Natriumcarbonatlösung zugibt, mit Methylenchlorid extrahiert und die Methylenchloridlösung trocknet und einengt. Das so erhaltene 2-Chlormethyl--3-methyl-4-methoxypyridin wird als Rohprodukt direkt weiterverarbeitet.
d) Eine Lösung von 690 mg rohem 2-Chlormethyl-3-methyl-4-- methoxypyridin und 400 mg 5,7-Dihydro-2-mercapto-5,5,7,7-- tetramethylindeno[5,6-d]imidazol-6(lH)-on in 40 ml abs. Aceton wird mit 1,9 g fein gemahlenem Kaliumcarbonat versetzt, worauf bei Raumtemperatur unter Argon während 18 Stunden gerührt wird. Nach Einengen des Gemisches im Vakuum chromatographiert man den Rückstand an Kieselgel mit ^{g}ethylenchlorid/Essigester (10:1) als Elutionsmittel, wobei die Methode der Mitteldruck-Flashchromatographie verwendet und der Druck mit Stickstoffgas erzeugt wird. Durch Umkristallisieren aus Essigester/Aether erhält man 5,7-Dihy- dro-2-[[(4-methoxy-3-methyl-2-pyridyl)methyl]thio]-5,5,7,7-- tetramethylindeno[5,6-d]imidazol-6(lH)-on vom Schmelzpunkt 218-220°.

### Beispiel 10

a) Zu 500 ml einer 5%igen Lösung von Methyllithium in Aether tropft man bei Raumtemperatur unter Argon 1200 ml Aether, anschliessend 35,6 g 3,5-Lutidin (3,5-Dimethylpyridin) und schliesslich 400 ml Toluol zu. Der Aether wird vollständig abdestilliert, worauf die Lösung während 4 Stunden bei 100° gerührt wird. Unter Methanol/Eis-Kühlung gibt man dann portionenweise Eis zu, bis keine Wärmeentwicklung mehr eintritt. Die Toluol-Phase wird vom ausgefallenen Festkörper abgetrennt und mit 66 ml halbkonzentrierter Salzsäure extrahiert. Die abgetrennte wässrige Phase stellt man mit 3N Natronlauge unter Kühlung auf ein pH von etwa 10 und extrahiert sie zweimal mit 300 ml Aether. Die Aetherextrakte werden über Natriumsulfat getrocknet und eingedampft. Der Rückstand wird im Vakuum bei 20 mm/72-74° destilliert: man erhält 2,3,5-Collidin (2,3,5-Trimethylpyridin), welches gemäss Gaschromatogramm eine 99,15%ige Reinheit aufweist.
b) Zu 246,4 g 2,3,5-Collidin und 2400 ml Eisessig werden bei Raumtemperatur 420 ml 30%iges Wasserstoffperoxyd zugetropft. Die Lösung wird über Nacht bei 80° gerührt. Dann kühlt man das Reaktionsgemisch auf 40° ab, gibt nochmals 420 ml 30%iges Wasserstoffperoxyd zu und erhitzt für weitere 24 Stunden auf 80°. Nach Eindampfen im Vakuum wird der Rückstand in 300 ml Wasser gelöst, worauf die Lösung unter Kühlung mit konz. Natronlauge basisch gestellt, mit Natriumchlorid gesättigt und dreimal mit 1 1 Methylenchlorid extrahiert wird. Die organischen Phasen werden über Natriumsulfat getrocknet und im Vakuum eingedampft. Der Rückstand wird aus Aether/Petroläther kristallisiert; man erhält 2,3,5-Trimethyl-pyridin-1-oxid vom Schmelzpunkt 42-44°.
c) Zu 210 ml konz. Schwefelsäure tropft man unter Kühlung 65 ml rauchende Salpetersäure (d=₁,₅) zu. Anschliessend werden bei 0-5° portionenweise 96,5 g 2,3,5-Trimethylpyri- din-1-oxid zugegeben, worauf das Gemisch 1 Stunde bei Raumtemperatur gerührt, dann innerhalb von 3 Stunden auf 90° aufgeheizt und über Nacht bei dieser Temperatur belassen wird. Nach dem Abkühlen wird die Lösung auf 1,5 kg Eis gegossen, worauf mit konz. Natronlauge auf pH 3 gestellt und dreimal mit 500 ml Methylenchlorid extrahiert wird. Die vereinigten organischen Phasen werden mit 1 1 Wasser gewaschen, über Natriumsulfat getrocknet und im Vakuum eingedampft. Der Rückstand wird aus Aether/Petroläther kristallisiert, und man erhält 2,3,5-Trimethyl-4-nitropyridin-1-- oxid vom Schmelzpunkt 76-78°.
d) 22,6 g Natrium werden unter Argon in 4 1 Methanol gelöst. Dann gibt man portionenweise 120 g 2,3,5-Trimethyl--4-nitropyridin-l-oxid zu und lässt die Lösung über Nacht unter Rückfluss kochen. Man stellt das pH unter Kühlung mittels 5N Chlorwasserstoff in Essigester auf 7 und dampft dann das Gemisch im Vakuum ein. Man nimmt den Rückstand in 1,5 1 Methylenchlorid auf, filtriert die Lösung durch Kieselgur, das noch mit 0,5 1 Methylenchlorid nachgewaschen wird, dampft die vereinigten Filtrate im Vakuum ein und kristallisiert den Rückstand aus Petroläther. Man erhält 4-Methoxy-2,3,5,-trimethylpyridin-1-oxid vom Schmelzpunkt 48-50°.
e) Zu einer Lösung von 81,5 g 4-Methoxy-2,3,5-trimethyl- pyridin-l-oxid in 290 ml Chloroform werden bei Raumtemperatur 215 ml Acetanhydrid zugetropft. Nach 4 Stunden Kochen unter Rückfluss dampft man die Lösung ein, löst den Rückstand in 200 ml Toluol und dampft erneut ein. Der Rückstand wird in 500 ml Essigester aufgenommen und dreimal mit 250 ml gesättigter Natriumbicarbonatlösung ausgeschüttelt. Die organische Phase wird über Natriumsulfat getrocknet und im Vakuum eingedampft. Das als Rückstand verbleibende Rohprodukt wird an 400 g Kieselgel mit Aether chromatographiert. Man erhält (4-Methoxy-3,5-dimethyl-2-pyridyl)-methylacetat in Form eines Oels.
f) 94,9 g (4-Methoxy-3,5-dimethyl-2-pyridyl)methylacetat werden in 570 ml Aethanol gelöst. Dann tropft man bei 0° 285 ml 3N Natronlauge zu und rührt das Gemisch 3 Stunden bei Raumtemperatur. Anschliessend wird das Aethanol im Vakuum entfernt, worauf man die zurückgebliebene wässrige Lösung dreimal mit 300 ml Methylenchlorid extrahiert. Die organischen Auszüge werden über Natriumsulfat getrocknet und im Vakuum eingedampft. Der Rückstand wird aus Petroläther kristallisiert, und man erhält 4-Methoxy-3,5-dimethyl-2-pyridylmethanol vom Schmelzpunkt 49-51°.
g) Zu 38 ml Thionylchlorid in 400 ml Methylenchlorid werden bei 0° 75,8 g 4-Methoxy-3,5-dimethyl-2-pyridylmethanol, gelöst in 200 ml Methylenchlorid, zugetropft. Nach 16-stündigem Rühren bei Raumtemperatur tropft man unter Kühlung 1800 ml Aether zu und rührt das Gemisch 2 Stunden bei Raumtemperatur weiter. Die ausgefallenen Kristalle werden abgenutscht und mit Aether gewaschen. Man erhält 2-(Chlormethyl)-4-methoxy-3,5-dimethylpyridin-hydrochlorid von Schmelzpunkt 130-131°.
h) 18,0 g (69,2 mMol) 5,7-Dihydro-2-mercapto-5,5,7,7-- tetramethylindeno[5,6-d]imidazol-6(1H)-on werden in 400 ml Alkohol suspendiert und unter Eiskühlung mit 15,6 g (70,2 mMol) 2-Chlormethyl-4-methoxy-3,5-dimethylpyridin-hydrochlorid versetzt. Hernach tropft man eine Lösung von 5,6 g Natriumhydroxyd in 150 ml Wasser zu, lässt das Gemisch über Nacht am Rückfluss kochen und dampft es anschliessend in Vakuum zur Trockene ein. Der Rückstand wird in 1000 ml Methylenchlorid gelöst; die Lösung wird zuerst mit 500 ml 1,5N Natronlauge und dann mit 3 x 500 ml Wasser gewaschen, über Natriumsulfat getrocknet und in Vakuum eingedampft. Das Rohprodukt wird an 300 g Kieselgel mit Essigester/Methylenchlorid (1:1) als Eluierungsmittel gereinigt. Kristallisation aus Methylenchlorid/Petroläther ergibt 5,7-Dihydro-2-[[(4-methoxy-3,5-dimethyl-2-pyridyl)methyl]-thio]-5,5,7,7-tetramethylindeno[5,6-d]imidazol-6(1H)-on vom Schmelzpunkt 166-168°.
i) 2,6 g 5,7-Dihydro-2-[[(4-methoxy-3,5-dimethyl-2-pyridyl)methyl]thio]-5,5,7,7-tetramethylindeno[5,6-d]imidazol--6(lH)-on werden in 50 ml heissem Methanol gelöst und mit 50 al einer 4N Lösung von Chlorwasserstoff in Methanol 10 Minuten unter Rückfluss gekocht. Nach Einengen im Vakuum kristallisiert man mit Aether und erhält 5,7-Dihydro-2--[[(4-methoxy-3,5-dimethyl-2-pyridyl)methyl]thio]-5,5,7,7-- tetramethylindeno[5,6-d]imidazol-6(lH)-on-dihydrochlorid vom Schmelzpunkt 165-170°.

### Beispiel 11

8,3 g 5,7-Dihydro-2-[[(4-methoxy-3,5-dimethyl-2-pyridyl)methyl]thio]-5,5,7,7-tetramethylindeno[5,6-d]imidazol--6(lH)-on werden in 1000 ml Methylenchlorid gelöst und mit einem Eis/Methanol-Bad auf -10° gekühlt. Dann werden innerhalb von 25 Minuten 4,3 g aus Methylenchlorid/Petroläther umkristallisierte m-Chlorperbenzoesäure eingetragen. Man rührt die Lösung bei -10° noch 45 Minuten weiter und giesst sie dann in ein Gemisch von 100 ml 2N Natriumcarbonatlösung und Eis. Die wässrige Phase wird zweimal mit 300 ml Methylenchlorid extrahiert. Die vereinigten organischen Phasen werden dreimal mit 200 ml Wasser neutralgewaschen, über Natriumsulfat getrocknet und im Vakuum bei 35° auf ein Volumen von 150 ml eingeengt. Bei Zugabe von Petroläther kristallisiert 5,7-Dihydro-2-[[(4-methoxy-3,5-dimethyl-2-- pyridyl)methyl]sulfinyl]-5,5,7,7-tetramethylindeno[5,6-d]imidazol-6(1H)-on vom Schmelzpunkt 192-194°.

### Beispiel 12

5 g 5,7-Dihydro-2-[[(4-methoxy-3,5-dimethyl-2-pyridyl)-methyl]thio]-5,5,7,7-tetramethylindeno[5,6-d]imidazol-6(lH)-- on und 40 g Hydroxylamin-hydrochlorid werden in 250 ml Methanol gelöst und über Nacht unter Argon am Rückfluss gekocht. Nach Abdampfen von ca. 200 ml Methanol im Vakuum wird die so erhaltene Suspension in einen vorher mit Argon begasten Scheidetrichter auf Eis gegossen. Man stellt das Gemisch mit gesättigter Natriumbicarbonatlösung neutral und extrahiert es fünfmal mit Chloroform/Methanol (3:1). Die organischen Extrakte werden über Natriumsulfat getrocknet und im Vakuum eingedampft. Den hellrosa Rückstand löst man in ca. 1 1 Essigsäureäthylester auf und filtriert die Lösung durch 30 g Kieselgel (in Essigester zubereitet). Bein Einengen des Eluats in Vakuum kristallisiert 5,7-Dihy- dro-2-[[(4-methoxy-3,5-dimethyl-2-pyridyl)methyl]thio]-5,5,-7,7-tetramethylindeno[5,6-d]imidazol-6(1H)-on-oxim aus; Smp. 233-235°.

### Beispiel 13

1 g 5,7-Dihydro-2-[[(4-methoxy-3,5-dimethyl-2-pyridyl)-methyl]thio]-5,5,7,7-tetramethylindeno[5,6-d]imidazol-6(1H)-- on-oxim wird in 50 ml Methylenchlorid und 5 ml Methanol gelöst und auf -20° gekühlt. Dann werden innerhalb 5 Minuten 0,6 g aus Methylenchlorid/Petroläther umkristallisierte m-Chlorperbenzoesäure eingetragen. Man rührt die Lösung bei dieser Teaperatur noch 30 Minuten weiter und giesst sie dann in ein Gemisch von 20 ml 2N Natriumcarbonatlösung und Eis. Die wässrige Phase wird zweimal mit 100 ml Methylenchlorid extrahiert. Die vereinigten organischen Phasen werden dreimal mit 50 ml Wasser neutralgewaschen, über Natriumsulfat getrocknet und im Vakuum bei 35° auf ein Volumen von 25 ml eingeengt. Bei Zugabe von Petroläther erfolgt Kristallisation von 5,7-Dihydro-2-[[(4-methoxy-3,5-dimethyl-2-pyridyl)methyl]sulfinyl]-5,5,7,7-tetramethylindeno-[5.6-d]imidazol-6(1H)-on-oxim; Smp. 215-217°.

### Beispiel 14

5 g 5,7-Dihydro-2-[[(4-methoxy-3,5-dimethyl-2-pyridyl)-methyl]thio]-5,5,7,7-tetramethylindeno[5,6-d]imidazol-6(1H)― on und 40 g O-Methylhydroxylamin-hydrochlorid werden in 250 ml Methanol 16 Stunden unter Argon an Rückfluss gekocht. Nach dem Entfernen des Lösungsmittels in Vakuum wird der Rückstand zwischen Methylenchlorid und Wasser verteilt, und die wässrige Phase wird dreimal mit Methylenchlorid extrahiert. Die vereinigten organischen Auszüge stellt man in Gegenwart von Eis und unter Argon-Begasung mit gesättigter Natriumbicarbonatlösung neutral. Nach mehrmaliger Extraktion mit Methylenchlorid werden die organischen Phasen über Natriumsulfat getrocknet und im Vakuum eingedampft. Eine rote Verunreinigung wird durch Chromatograhie an Kieselgel mit Essigsäureäthylester entfernt. Die Trennung des Reaktionsproduktes vom Ausgangsmaterial erfolgt an Kieselgel mit Toluol/Methylisobutylketon/Pyridin (80:18:2) als Eluierungsmittel. Die Reinheit der Fraktionen wird dünnschichtchromatographisch mit dem System Essigester/Methylisobutylketon/Pyridin (80:18:2) geprüft. Durch Kristallisation aus Aceton/Wasser erhält man 5,7-Dihydro-2-[[(4-methoxy-3,5-- dimethyl-2-pyridyl)methyl]thio]-5,5,7,7-tetramethylindeno-[5,6-d]imidazol-6(1H)-on-O-methyloxim vom Schmelzpunkt 110-112°.

### Beispiel 15

2,6 g 5,7-Dihydro-2-[[(4-methoxy-3,5-dimethyl-2-pyridyl)methyl]thio]-5,5,7,7-tetramethylindeno[5,6-d]imidazol-6(lH)-on-O-methyloxim werden in 250 ml Methylenchlorid gelöst und mit einem Trockeneis/Aceton-Bad auf -30° gekühlt. Dann werden innerhalb von 10 Minuten 1,26 g aus Methylenchlorid/Petroläther umkristallisierte m-Chlorperbenzoesäure eingetragen. Man rührt die Lösung bei dieser Tempratur noch 50 Minuten weiter, giesst sie dann in ein Gemisch von 50 ml 2N Natriumcarbonatlösung und Eis und extrahiert die wässrige Phase zweimal mit 150 ml Methylenchlorid. Die vereinigten organischen Phasen werden dreimal mit 100 ml Wasser neutralgewaschen, über Natriumsulfat getrocknet und im Vakuum bei 35° auf ein Volumen von 50 ml eingeengt. Bei Zugabe von Petroläther erfolgt Kristallisation von 5,7-Dihy- dro-2-[[(4-methoxy-3,5-dimethyl-2-pyridyl)methyl]sulfinyl]-5,5,7,7-tetramethylindeno[5,6-d]imidazol-6(lH)-on-O-methyloxim vom Schmelzpunkt 173-175°.

### Beispiel 16

a) 4,2 g Natrium werden in 780 ml Aethanol unter Argon gelöst. Dann gibt man portionenweise 22,3 g 2,3,5-Trimethyl--4-nitropyridin-1-oxid zu und lässt die Lösung über Nacht unter Rückfluss kochen. Man stellt das pH unter Kühlung mittels 5N Chlorwasserstoff in Essigester auf 7 und dampft dann das Gemisch im Vakuum ein. Man nimmt den Rückstand in 0,5 1 Methylenchlorid auf, filtriert die Lösung durch Kieselgur, das noch mit 200 ml Methylenchlorid nachgewaschen wird, dampft die vereinigten Filtrate im Vakuum ein und kristallisiert den Rückstand aus Petroläther. Man erhält 4-Aethoxy-2,3,5-trimethylpyridin 1-oxid vom Schmelzpunkt 59-61°.
b) Zu einer Lösung von 31,3 g 4-Aethoxy-2,3,5-trimethyl- pyridin-1-oxid in 100 ml Chloroform werden bei Raumtemperatur 75 al Acetanhydrid zugetropft. Nach 16 Stunden Kochen unter Rückfluss dampft man die Lösung ein, löst den Rückstand in 100 ml Toluol und dampft erneut ein. Der Rückstand wird in 250 ml Essigester aufgenommen, worauf dreimal mit 100 ml gesättigter Natriumbicarbonatlösung ausgeschüttelt wird. Die organische Phase wird über Natriumsulfat getrocknet und im Vakuum eingedampft. Das Rohprodukt wird an 170 g Kieselgel mit Aether chromatographiert. Man erhält (4-Aethoxy-3,5-dimethyl-2-pyridyl)methylacetat in Form eines Oels.
c) 32,9 g (4-Aethoxy-3,5-dimethyl-2-pyridyl)methylacetat werden in 190 ml Aethanol gelöst. Dann tropft man bei 0° 95 ml 3N Natronlauge zu und rührt das Gemisch 3 Stunden bei Raumtemperatur weiter. Anschliessend entfernt man das Aethanol im Vakuum und extrahiert die zurückgebliebene wässrige Lösung dreimal mit 200 ml Methylenchlorid. Die organischen Auszüge werden über Natriumsulfat getrocknet und im Vakuum eingedampft. Der Rückstand kristallisiert aus Petroläther, und man erhält 4-Aethoxy-3,5-dimethyl-2-pyri- dylmethanol vom Schmelzpunkt 58-59°.
d) Zu 10 ml Thionylchlorid in 220 ml Methylenchlorid werden bei 0° 21,0 g 4-Aethoxy-3,5-dimethyl-2-pyridylmethanol, gelöst in 110 ml Methylenchlorid, zugetropft. Nach 16 Stunden Rühren bei Raumtemperatur tropft man unter Kühlung 890 ml Aether zu und rührt das Gemisch 2 Stunden bei Raumtemperatur weiter. Die ausgefallenen Kristalle werden abgenutscht und mit Aether gewaschen. Man erhält 2-Chlormethyl-4-äthoxy-3,5-dimethylpyridin-hydrochlorid vom Schmelzpunkt 156-158°.
e) 7,8 g (30 mMol) 5,7-Dihydro-2-mercapto-5,5,7,7-tetra- methylindeno[5,6-d]imidazol-6(lH)-on werden in 200 ml Alkohol suspendiert und unter Eiskühlung mit 7,1 g (30 mMol) 2-Chlormethyl-4-äthoxy-3,5-dimethylpyridin-hydrochlorid versetzt. Hernach tropft man eine Lösung von 2,4 g Natriumhydroxyd in 100 ml Wasser zu, lässt das Gemisch über Nacht am Rückfluss kochen und dampft es anschliessend im Vakuum zur Trockne ein. Der Rückstand wird in 500 ml Methylenchlorid gelöst. Die Lösung wird zuerst mit 250 ml 1,5N Natronlauge und dann mit 3 x 250 ml Wasser gewaschen, über Natriumsulfat getrocknet und im Vakuum eingedampft. Das Rohprodukt wird an 150 g Kieselgel mit Essigester/Methylenchlorid (1:1) als Eluierungsmittel gereinigt. Kristallisation aus Methylenchlorid/Petroläther ergibt 5,7-Dihydro-2--[[(4-äthoxy-3,5-dimethyl-2-pyridyl)methyl]thio]-5,5,7,7-- tetramethylindeno[5,6-d]imidazol-6(lH)-on vom Schmelzpunkt 179-180°.

### Beispiel 17

7,3 g 5,7-Dihydro-2-[[(4-äthoxy-3,5-dimethyl-2-pyridyl)methyl]thio]-5,5,7,7-tetramethylindeno[5,6-d]imidazol--6(lH)-on werden in 750 ml Methylenchlorid gelöst und mit einem Eis/Methanol-Bad auf -10° gekühlt. Dann werden innerhalb von 30 Minuten 3,7 g aus Methylenchlorid/Petroläther umkristallisierte m-Chlorperbenzoesäure eingetragen. Man rührt die Lösung bei dieser Temperatur noch 75 Minuten weiter und giesst sie dann in ein Gemisch von 100 ml 2N Natriumcarbonatlösung und Eis. Die wässrige Phase wird zweimal mit 300 ml Methylenchlorid extrahiert. Die vereinigten organischen Phasen werden dreimal mit 200 ml Wasser neutralgewaschen. über Natriumsulfat getrocknet und im Vakuum bei 35° auf ein Volumen von 120 ml eingeengt. Bei Zugabe von Petroläther erfolgt Kristallisation von 5,7-Dihydro-2--[[(4-äthoxy-3,5-dimethyl-2-pyridyl)methyl]sulfinyl]-5,5,7,7-- tetramethylindeno[5,6-d]imidazol-6(1H)-on; Smp. 185-187°.

### Beispiel 18

a) Zu 321,5 g 2,5-Lutidin (2,5-Dimethylpyridin) in 1800 ml Eisessig werden bei Raumtemperatur 400 ml 30%iges Wasserstoffperoxyd zugetropft. Die Lösung wird über Nacht bei 80° gerührt, dann auf 40° abgekühlt, nochmals mit 400 ml 30%- igem Wasserstoffperoxyd versetzt und für weitere 24 Stunden auf 80° erhitzt. Nach Eindampfen im Vakuum wird der Rückstand in 300 ml Wasser gelöst. Die Lösung wird unter Kühlung mit konz. Natriumhydroxydiösung basisch gestellt, mit Natriumchlorid gesättigt und dreimal mit 1 1 Methylenchlorid extrahiert. Die organischen Auszüge werden über Natriumsulfat getrocknet und im Vakuum eingedampft. Man erhält 2,5-Dimethylpyridin-1-oxid in Form eines Oels.
b) Zu 840 al kohz. Schwefelsäure tropft man unter Kühlung 260 ml rauchende Salpetersäure (d=1,5) zu. Anschliessend werden bei 0-5° portionenweise 348,2 g 2,5-Dimethylpyridin-l-oxid zugegeben. Das Gemisch wird 1 Stunde bei Raumtemperatur gerührt, dann innerhalb von 3 Stunden auf 90° erhitzt, über Nacht bei dieser Temperatur belassen und nach dem Abkühlen auf 6 kg Eis gegossen. Man stellt mit konz. Natronlauge auf pH 3 und extrahiert dreimal mit 2 1 Methylenchlorid. Die vereinigten organischen Phasen werden mit 4 1 Wasser gewaschen, über Natriumsulfat getrocknet und im Vakuum eingedampft. Der Rückstand wird aus Methylenchlorid/Petroläther kristallisiert, und man erhält 2,5-Dimethyl-4-nitropyridin-l-oxid vom Schmelzpunkt 142-144°.
c) 12,2 g Natrium werden unter Argon in 2 1 Methanol gelöst, worauf man portionenweise 60 g 2,5-Dimethyl-4-nitro- pyridin-1-oxid zugibt und die Lösung über Nacht unter Rückfluss kochen lässt. Das pH wird unter Kühlung mit 5N Chlorwasserstoff in Essigester auf 7 gestellt, und das Gemisch wird im Vakuum eingedampft. Den Rückstand nimmt man in 1 1 Methylenchlorid auf, filtriert die Lösung durch Kieselgur, das noch mit 0,4 1 Methylenchlorid nachgewaschen wird, und dampft die vereinigten Filtrate im Vakuum ein. Der Rückstand kristallisiert aus Methylenchlorid/Petroläther, und man erhält 4-Methoxy-2,5-dimethylpyridin-1-oxid vom Schmelzpunkt 99-101°.
d) Zu einer Lösung von 19,9 g 4-Methoxy-2,5-dimethylpyridin-1-oxid in 75 ml Chloroform werden bei Raumtemperatur 55 ml Acetanhydrid zugetropft. Nach 2-stündigem Kochen unter Rückfluss dampft man die Lösung ein, löst den Rückstand in 100 ml Toluol und dampft erneut ein. Der Rückstand wird in 100 ml Essigester aufgenommen, und die Lösung wird dreimal mit 50 ml gesättigter Natriumbicarbonatlösung ausgeschüttelt. Die organische Phase wird über Natriumsulfat getrocknet und im Vakuum eingedampft. Das Rohprodukt wird an 100 g Kieselgel mit Aether chromatographiert, und man erhält (4-Methoxy-5-methyl-2-pyridyl)methylacetat in Form eines Oels.
e) 47,8 g (4-Methoxy-5-methyl-2-pyridyl)methylacetat werden in 330 ml Aethanol gelöst. Dann tropft man bei 0° 165 ml 3N Natronlauge zu und rührt das Gemisch 3 Stunden bei Raumtemperatur weiter. Anschliessend wird das Aethanol im Vakuum entfernt, worauf die zurückgebliebene wässrige Lösung dreimal mit 250 ml Methylenchlorid extrahiert wird. Die organischen Auszüge werden über Natriumsulfat getrocknet und im Vakuum eingedampft. Der Rückstand kristallisiert aus Petroläther. und man erhält 4-Methoxy-5-methyl-2-pyri- dylmethanol vom Schmelzpunkt 101-103°.
f) Zu 17 ml Thionylchlorid in 360 ml Methylenchlorid werden bei 0° 28,1 g 4-Methoxy-5-methyl-2-pyridylmethanol, gelöst in 180 ml Methylenchlorid, zugetropft. Nach 16 Stunden Rühren bei Raumtemperatur tropft man unter Kühlung 1400 ml Aether zu und rührt das Gemisch 2 Stunden bei Raumtemperatur weiter. Die ausgefallenen Kristalle werden abgenutscht und mit Aether gewaschen. Man erhält 2-(Chlormethyl)-4-- methoxy-5-methylpyridin-hydrochlorid vom Schmelzpunkt 149--151°.
g) 7,8 g (30 mMol) 5,7-Dihydro-2-mercapto-5,5,7.7-tetra- methylindeno[5,6-d]imidazol-6(1H)-on werden in 200 ml Alkohol suspendiert und unter Eiskühlung mit 6,3 g (30 mMol) 2-Chlormethyl-4-methoxy-5-methylpyridin-hydrochlorid versetzt. Hernach tropft man eine Lösung von 2,4 g Natriumhydroxyd in 100 ml Wasser zu, lässt das Gemisch über Nacht am Rückfluss kochen und dampft es anschliessend im Vakuum zur Trockne ein. Der Rückstand wird in 500 ml Methylenchlorid gelöst. Die Lösung wird zuerst mit 250 ml 1,5N Natronlauge und dann mit 3 x 250 ml Wasser gewaschen, über Natriumsulfat getrocknet und im Vakuum eingedampft. Das Rohprodukt reinigt man an 150 g Kieselgel mit Essigester/Methylenchlorid (1:1) als Eluierungsmittel. Kristallisation aus Methylenchlorid/Petroläther ergibt 5,7-Dihydro-2-[[(4-methoxy--5-methyl-2-pyridyl)methyl]thio]-5,5,7.7-tetramethylindeno-[5,6-d]imidazol-6(1H)-on vom Schmelzpunkt 204-205°.

### Beispiel 19

9,5 g 5,7-Dihydro-2-[[(4-methoxy-5-methyl-2-pyridyl)-methyl]thio]-5,5,7,7-tetramethylindeno[5,6-d]imidazol-6(1H)-- on werden in 1000 ml Methylenchlorid gelöst und mit einem Eis/Methanol-Bad auf -10° gekühlt. Dann werden innerhalb von 30 Minuten 4,7 g aus Methylenchlorid/Petroläther umkristallisierte m-Chlorperbenzoesäure eingetragen. Man rührt die Lösung bei -10° noch 120 Minuten weiter und giesst sie dann in ein Gemisch von 150 ml 2N Natriumcarbonatlösung und Eis ein. Die wässrige Phase wird zweimal mit 300 ml Methylenchlorid extrahiert. Die vereinigten organischen Phasen werden dreimal mit 200 ml Wasser neutralgewaschen, über Natriumsulfat getrocknet und im Vakuum bei 35° auf ein Volumen von 150 ml eingeengt. Bei Zugabe von Petroläther erfolgt Kristallisation von 5,7-Dihydro-2--[[(4-meth4xy-5-methyl-2-pyridyl)methyl]sulfinyl]-5,5,7,7-- tetramethylindeno[5,6-d]imidazol-6(lH)-on; Smp. 191-193°.

### Beispiel 20

a) 13,5 g Natrium werden unter Argon in 2300 ml Aethanol gelöst. Dann gibt man portionenweise 60 g 2,5-Dimethyl-4-nitropyridin-1-oxid zu und lässt die Lösung über Nacht unter Rückfluss kochen. Das pH wird unter Kühlung mit 5N Chlorwasserstoff in Essigester auf 7 gestellt, worauf das Gemisch im Vakuum eingedampft wird. Man nimmt den Rückstand in 1 1 Methylenchlorid auf, filtriert die Lösung durch Kieselgur, das noch mit 500 ml Methylenchlorid nachgewaschen wird, und dampft die vereinigten Filtrate im Vakuum ein. Das Rohprodukt wird an 300 g Kieselgel mit Methylenchlorid/Methanol (95:5) chromatographiert. Nach Kristallisation aus Aether/Petroläther erhält man 4-Aethoxy-2,5-dimethylpyridin-l-oxid vom Schmelzpunkt 65-67°.
b) Zu einer Lösung von 7,8 g 4-Aethoxy-2,5-dimethylpyridin-l-oxid in 30 ml Chloroform werden bei Raumtemperatur 20 ml Acetanhydrid zugetropft. Nach 3-stündigem Kochen unter Rückfluss dampft man die Lösung ein, löst den Rückstand in 50 ml Toluol und dampft erneut ein. Der Rückstand wird in 50 ml Essigester aufgenommen, worauf die Lösung dreimal mit 20 ml gesättigter Natriumbicarbonatlösung ausgeschüttelt wird. Die organische Phase wird über Natriumsulfat getrocknet und im Vakuum eingedampft. Das Rohprodukt wird an 50 g Kieselgel mit Aether chromatographiert, und man erhält (4-Aethoxy-5-methyl-2-pyridyl)methylacetat in Form eines Oels.
c) 7,4 g (4-Aethoxy-5-methyl-2-pyridyl)methylacetat werden in 46 ml Aethanol gelöst. Dann tropft man 23 ml 3N Natronlauge zu und rührt das Gemisch 3 Stunden bei Raumtemperatur weiter. Anschliessend wird das Aethanol im Vakuum entfernt, worauf man die zurückgebliebene wässrige Lösung dreimal mit 100 ml Methylenchlorid extrahiert. Die organischen Auszüge werden über Natriumsulfat getrocknet und im Vakuum eingedampft. Der Rückstand kristallisert aus Petroläther, und man erhält 4-Aethox^{y-}5-methyl-2-pyridylmethanol vom Schmelzpunkt 99-101°.
d) Zu 2,4 ml Thionylchlorid in 60 ml Methylenchlorid werden bei 0° 4,7 g 4-Aethoxy-5-methyl-2-pyridylmethanol. gelöst in 30 ml Methylenchlorid, zugetropft. Nach 16 Stunden Rühren bei Raumtemperatur tropft man unter Kühlung 400 ml Aether zu und rührt das Gemisch 2 Stunden bei Raumtemperatur weiter. Die ausgefallenen Kristalle werden abgenutscht und mit Aether gewaschen. Man erhält 2-Chlormethyl-4-- äthoxy-5-methylpyridin-hydrochlorid vom Schmelzpunkt 144--146°.
e) 6,1 g (23,4 mMol) 5,7-Dihydro-2-mercapto-5,5,7,7-tetra- methylindeno[5,6-d]imidazol-6(1H)-on werden in 100 ml Alkohol suspendiert und unter Eiskühlung mit 5,2 g (23,4 mMol) 2-Chlormethyl-4-äthoxy-5-methylpyridin-hydrochlorid versetzt. Hernach tropft man eine Lösung von 1,9 g Natriumhydroxyd in 50 ml Wasser zu, lässt das Gemisch über Nacht am Rückfluss kochen und dampft es anschliessend im Vakuum zur Trockne ein. Der Rückstand wird in 300 ml Methylenchlorid gelöst. Die Lösung wird zuerst mit 200 ml 1,5N Natronlauge und dann mit 3 x 200 ml Wasser gewaschen, über Natriumsulfat getrocknet und im Vakuum eingedampft. Das Rohprodukt reinigt man an 120 g Kieselgel mit Essnigester/Methylenchlorid (1:1) als Eluierungsmittel. Kristallisation aus Methylenchlorid/Petroläther ergibt 2-[[(4-Aethoxy-5-methyl-2-- pyridyl)methyl]thio]-5,7-dihydro-5,5,7,7-tetramethylindeno-[5,6-d]imidazol-6(lH)-on vom Schmelzpunkt 187-189°.

### Beispiel 21

6,1 g 2-[[(4-Aethoxy-5-methyl-2-pyridyl)methyl]thio]--5,7-dihydro-5,5,7,7-tetramethylindeno[5,6-d]imidazol-6(1H)-on werden in 300 ml Methylenchlorid gelöst und mit einem Trockeneis/Aceton-Bad auf -30° gekühlt. Dann werden innerhalb von 30 Minuten 4,0 g aus Methylenchlorid/Petroläther umkristallisierte m-Chlorperbenzoesäure eingetragen. Man rührt die Lösung bei -10° noch 120 Minuten weiter und giesst sie dann in ein Gemisch von 100 ml 2N Natriumcarbonatlösung und Eis. Die wässrige Phase wird zweimal mit 300 ml Methylenchlorid extrahiert. Die vereinigten organischen Phasen werden dreimal mit 200 ml Wasser neutralgewaschen, über Natriumsulfat getrocknet und im Vakuum bei 35° auf ein Volumen von 120 ml eingeengt. Bei Zugabe von Petroläther erfolgt Kristallisation von 2-[[(4-Aethoxy-5-- methyl-2-pyridyl)methyl]sulfinyl)-5,7,-dihydro-5,5,7,7-tetra- methylindeno[5,6-d]imidazol-6(lH)-on, Smp. 205-207°.

### Beispiel 22

a) Eine Lösung von 7,3 g (0,039 Mol) 2-Chlormethyl-3-- methyl-4-nitro-pyridin und 7,5 g (0,039 Mol) 5H-1,3-Dioxo- lo[4.5-f]benzimidazol-6-thiol in 200 ml abs. Aceton wird mit 8 g fein gemahlenem Kaliumcarbonat versetzt, worauf bei Raumtemperatur unter Argon 2 Stunden gerührt wird. Die Mischung wird auf Eis gegossen, und die entstandenen Kristalle werden abfiltriert, gründlich mit Wasser gewaschen und in Acetonitril gelöst. Die erhaltene Lösung wird heiss filtriert. Beim Abkühlen des Filtrats kristallisiert 6-[[(3-Methyl-4-nitro-2-pyridyl)methyl]thio]-5H-1,3-dioxolo-[4,5-f]benzimidazol vom Schmelzpunkt 204-205° (Zersetzung).
b) Eine Lösung von 500 mg (1,45 mMol) 6-[[(3-Methyl-4-- nitro-2-pyridyl)methyl)thio]-5H-1,3-dioxolo[4,5-f)benzimidazol in 20 ml abs. Methanol wird mit 300 mg Natriummethylat versetzt, worauf unter Argon während 18 Stunden am Rückfluss gekocht wird. Die Reaktionsmischung wird mittels Eisessig gepuffert und im Vakuum eingeengt. Der Rückstand wird mit Methylenchlorid/Natriumbicarbonatlösung versetzt, worauf man die organische Lösung trocknet und einengt. Durch Umkristallisieren aus Essigester erhält man 6-[[(4-Me- thoxy-3-methyl-2-pyridyl)methyl]thio]-5H-1,3-dioxolo[4,5-f]-benzimidazol vom Schmelzpunkt 215-220°.

### Beispiel 23

Eine Lösung von 330 mg (1 mMol) 6-[[(4-Methoxy-3-- methyl-2-pyridyl)methyl)thio]-5H-1,3-dioxolo[4,5-f]benzimidazol in 5 ml Chloroform wird unter Eiskühlung und Rühren portionenweise mit 200 mg (1,2 mMol) m-Chlorperbenzoesäure versetzt. Nach 15 Minuten wird die Reaktionsmischung mit 10%iger Natriumcarbonatlösung extrahiert, getrocknet und eingeengt. Der Rückstand wird an Kieselgel mit Methylenchlorid/Methanol (8,5:1,5) als Elutionsmittel chromatographiert, wobei die Methode der Mitteldruck-Flashchromatographie verwendet und der Druck mit Stickstoffgas erzeugt wird. Durch Umkristallisieren aus Aether erhält man 6-[[(4-Methoxy-3-methyl-2-pyridyl)methyl]sulfinyl]-5H-1,3-- dioxoloE4,5-f]benzimidazol vom Schmelzpunkt 185-186° (Zersetzung).

### Beispiel 24

a) 14,8 g (76,7 mMol) 5H-1.3-Dioxolo[4.5-f]benzimidazol--6-thiol werden in 300 ml Alkohol suspendiert und unter Eiskühlung mit 17,0 g (76,5 mMol) 2-Chlormethyl-4-methoxy--3,5-dimethylpyridin-hydrochlorid versetzt. Hernach tropft man eine Lösung von 6,0 g Natriumhydroxyd in 150 ml Wasser zu, lässt das Gemisch über Nacht am Rückfluss kochen und dampft es anschliessend im Vakuum zur Trockne ein. Der Rückstand wird in 1000 ml Methylenchlorid gelöst. Die Lösung wird zuerst mit 500 ml 1,5N Natronlauge und dann mit 3 x 500 ml Wasser gewaschen, über Natriumsulfat getrocknet und im Vakuum eingedampft. Das Rohprodukt reinigt man an 300 g Kieselgel mit Essigester/Methylenchlorid (1:1) als Eluierungsmittel. Kristallisation aus Methylenchlorid/Petroläther ergibt 6-[[(4-Methoxy-3,5-dimethyl-2-pyridyl)-methyl]thio]-5H-1,3-dioxolo[4,5-f]benzimidazol vom Schmelzpunkt 178-179°.
b) 1,3 g 6-[[(4-Methoxy-3,5-dimethyl-2-pyridyl)methyl]-thio]-5H-1,3-dioxolo[4,5-f]benzimidazol werden in 25 ml heissem Methanol gelöst und mit 40 ml einer 5N Lösung von Chlorwasserstoff in Essigester versetzt. Bei Zugabe von Aether kristallisiert 6-[[(4-Methoxy-3,5-dimethyl-2-pyridyl)methyl]thio]-5H-1,3-dioxolo[4,5-f]benzimidazol-dihydrochlorid vom Schmelzpunkt 208-210°.

### Beispiel 25

13,3 g 6-[[(4-Methoxy-3,5-dimethyl-2-pyridyl)methyl]-thio]-5H-1,3-dioxolo[4,5-f]benzimidazol werden in 300 ml Methylenchlorid gelöst und mit einem Eis/Methanol-Bad auf -10° gekühlt. Dann werden innerhalb von 30 Minuten 7,5 g aus Methylenchlorid/Petroläther umkristallisierte m-Chlorperbenzoesäure eingetragen. Man rührt die Lösung bei -10° noch 120 Minuten weiter und giesst sie dann in ein Gemisch von 300 ml 2N Natriumcarbonatlösung und Eis. Die wässrige Phase wird zweimal mit 300 ml Methylenchlorid extrahiert. Die vereinigten organischen Phasen werden dreimal mit 250 ml Wasser neutralgewaschen, über Natriumsulfat getrocknet und im Vakuum bei 35° auf ein Volumen von 150 ml eingeengt. Bei Zugabe von Petroläther erfolgt Kristallisation von 6-[[(4-Methoxy-3,5-dimethyl-2-pyridyl)methyl]sulfinyl]-5H-1,3-dioxolo[4,5-f]benzimidazol; Smp. 192-194°.

### Beispiel 26

5,82 g (30,0 mMol) 5H-1,3-Dioxolo[4,5-f]benzimidazol--6-thiol werden in 200 ml Alkohol suspendiert und unter Eiskühlung mit 7,1 g (30,1 mMol) 2-Chlormethyl-4-äthory-3.5-- dimethylpyridin-hydrochlorid versetzt. Hernach tropft man eine Lösung von 2,4 g Natriumhydroxyd in 100 ml Wasser zu, lässt das Gemisch über Nacht am Rückfluss kochen und dampft es anschliessend im Vakuum zur Trockne ein. Der Rückstand wird in 500 ml Methylenchlorid gelöst. Die Lösung wird zuerst mit 250 ml 1,5N Natronlauge und dann mit 3 x 300 ml Wasser gewaschen, über Natriumsulfat getrocknet und im Vakuum eingedampft. Das Rohprodukt reinigt man an 150 g Kieselgel mit Essigester/Methylenchlorid (1:1) als Eluierungsmittel. Kristallisation aus Acetonitril ergibt 6-[[(4-Aethoxy-3,5-dimethyl-2-pyridyl)methyl]thio]-5H-1,3-- dioxolo[4,5-f]benzimidazol vom Schmelzpunkt 184-185°.

### Beispiel 27

7,4 g 6-[[(4-Aethoxy-3,5-dimethyl-2-pyridyl)methyl]-thio]-5H-1,3-diozalo[4,5-f]benzimidazol werden in 1000 ml Methylenchlorid gelöst und mit Trockeneis/Aceton auf -30° gekühlt. Dann werden innerhalb von 10 Minuten 4,3 g aus Methylenchlorid/Petroläther umkristallisierte m-Chlorperbenzoesäure eingetragen. Man rührt die Lösung bei -30° noch 45 Minuten weiter und giesst sie dann in ein Gemisch von 100 ml 2N Natriumcarbonatlösung und Eis. Die wässrige Phase wird zweimal mit 400 ml Methylenchlorid extrahiert. Die vereinigten organischen Phasen werden dreimal mit 300 ml Wasser neutralgewaschen, über Natriumsulfat getrocknet und im Vakuum bei 35° auf ein Volumen von 100 ml eingeengt. Bei Zugabe von Petroläther erfolgt Kristallisation von 6-[[(4--Aethoxy-3,5-dimethyl-2-pyridyl)methyl]sulfinyl]-5H-1,3-dioxo- lo[4,5-f]benzimidazol, Smp. 197-198°.

### Beispiel 28

5,82 g (30.0 mMol) 5H-1,3-Dioxolo[4,5-f]benzimidazol--6-thiol werden in 200 ml Alkohol suspendiert und unter Eiskühlung mit 6,3 g (30,3 mMol) 2-Chlormethyl-4-methoxy-5-- methylpyridin-hydrochlorid versetzt. Hernach tropft man eine Lösung von 2,4 g Natriumhydroxyd in 100 ml Wasser zu, lässt das Gemisch über Nacht am Rückfluss kochen und dampft es anschliessend im Vakuum zur Trockne ein. Der Rückstand wird in 500 ml Methylenchlorid gelöst. Die Lösung wird zuerst mit 250 ml 1,5N Natronlauge und dann mit 3 x 300 ml Wasser gewaschen, über Natriumsulfat getrocknet und im Vakuum eingedampft. Das Rohprodukt reinigt man an 150 g Kieselgel mit Essigester/Methylenchlorid (1:1) als Eluierungsmittel. Kristallisation aus Methylenchlorid/Petroläther ergibt 6-[[(4-Methoxy-5-methyl-2-pyridyl)methyl]-thio]-5H-1,3-dioxolo[4,5-f]benzimidazol vom Schmelzpunkt 191-193°.

### Beispiel 29

4,3 g 6-[[(4-Methoxy-5-methyl-2-pyridyl)methyl]thio]--5H-l,3-dioxolo[4,5-f]benzimidazol werden in 100 ml Methylenchlorid gelöst und mit einem Eis/Methanol-Bad auf -10° gekühlt. Dann werden innerhalb von 30 Minuten 3,0 g aus Methylenchlorid/Petroläther umkristallisierte m-Chlorperbenzoesäure eingetragen. Man rührt die Lösung bei -10° noch 120 Minuten weiter und giesst sie dann in ein Gemisch von 100 ml 2N Natriumcarbonatlösung und Eis. Die wässrige Phase wird zweimal mit 100 ml Methylenchlorid extrahiert. Die vereinigten organischen Phasen werden dreimal mit 150 ml Wasser neutralgewaschen, über Natriumsulfat getrocknet und im Vakuum bei 35° auf ein Volumen von 50 ml eingeengt. Bei Zugabe von Petroläther erfolgt Kristallisation von 6-[[(4--Methoxy-5-methyl-2-pyridyl)methyl]sulfinyl]-5H-1,3-dioxolo-[4,5-f]benzimidazol; Smp. 182-184°.

### Beispiel 30

a) Eine Lösung von 500 mg (1,92 mMol) 5,7-Dihydro-2-mer- capto-5,5,7,7-tetramethylindeno[5,6-d]imidazol-6(1H)-on in 50 ml 1,2-Dichloräthan wird mit 790 mg Phosphortrichlorid versetzt und während 16 Stunden unter Rückfluss erhitzt. Nach dem Abkühlen wird das Gemisch auf Eis und Natriumcarbonat gegossen. Man extrahiert mit Methylenchlorid, trocknet die organische Phase und entfernt die Lösungsmittel am Rotationsverdampfer. Das als Rückstand verbleibende 5,7-Dihydro-2-chlor-5,5,7,7-tetramethylindeno[5,6-d]imidazol-6(lH)-on wird als Rohprodukt weiterverarbeitet.
b) Eine Lösung von 470 mg rohem 2-Chlormethyl-3-methyl-4--methoxypyridin, gelöst in 50 al Methylenchlorid, wird mit 5 ml Triäthylamin und 0,8 ml Thioessigsäure versetzt und über Nacht bei Raumtemperatur gerührt. Die Lösung wird auf Eis und Natriumcarbonat gegossen. Man extrahiert mit Methylenchlorid, trocknet die organische Phase und entfernt das Lösungsmittel. Das als Rückstand verbleibende 2-Acetyl- thiomethyl-3-methyl-4-methoxypyridin wird als Rohprodukt weiterverwendet.
c) Eine Lösung von 500 mg des obigen rohen 2-Acetylthio- methyl-3-methyl-4-methoxypyridins in 20 ml Methanol wird mit 5 ml Triäthylamin versetzt und über Nacht bei Raumtemperatur unter Stickstoff gerührt. Nach dem Entfernen des Lösungsmittels am Rotationsverdampfer verbleibt rohes 2-Thiomethyl-3-methyl-4-methoxypyridin als Rückstand, welches direkt weiterverarbeitet wird.
d) Eine Lösung von 500 mg des gemäss a) erhaltenen rohen 5,7-Dihydro-2-chlor-5,5,7,7 -tetramethylindeno[5.6-d]imidazol-6(lH)-on und 330 mg des gemäss c) erhaltenen rohen 2-Thiomethyl-3-methyl-4-methoxypyridins in 50 ml Aceton wird mit 2,5 g fein gemahlenem Kaliumcarbonat versetzt und während 18 Stunden bei Raumtemperatur unter Argon gerührt. Nach Filtration und Entfernen des Lösungsmittels wird der Rückstand an Kieselgel mit Methylenchlorid/Essigester (10:1, Mitteldruck-Flashchromatographie) chromatographiert. Durch Umkristallisation aus Essigester/Aether erhält man 5,7-Dihydro-2-[[(4-methoxy-3-methyl -2-pyridyl)methyl]thio] -5,5,7,7-tetramethylindeno[5,6-d]imidazol-6(lH)-on vom Schmelzpunkt 218-220°.

### Beispiel A

Verbindungen der Formel I können als Wirkstoffe zur Herstellung von Hartgelatinekapseln verwendet werden, deren Inhalt pro Kapsel folgende Zusammensetzung aufweist:

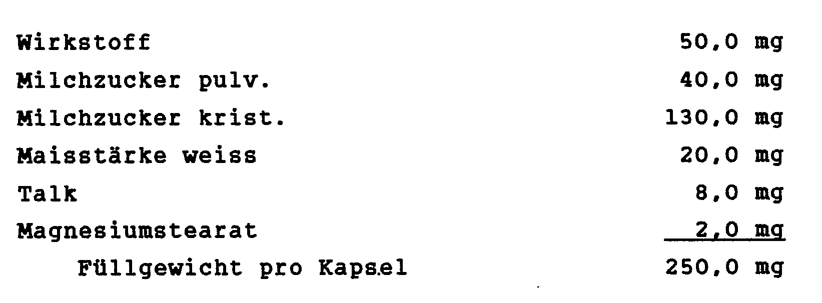

Der Wirkstoff und die Hilfsstoffe werden miteinander vermischt, und das Gemisch wird in Hartgelatinekapseln geeigneter Grösse abgefüllt. Erforderlichenfalls werden die Kapseln anschliessend mit einem magensaftresistenten Lack, bestehend aus Hydroxypropylmethylcellulosephthalat, versehen.

## Patentansprüche

1. Trieyclische Imidazolderivate der allgemeinen Formel 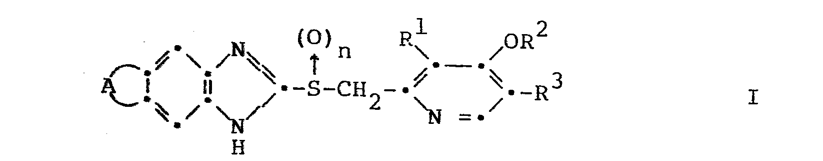 worin eines von R¹ und R³ niederes Alkyl und das andere Wasserstoff oder niederes Alkyl, R² niederes Alkyl, n die Zahl 0 oder 1. A einen Rest der Formel 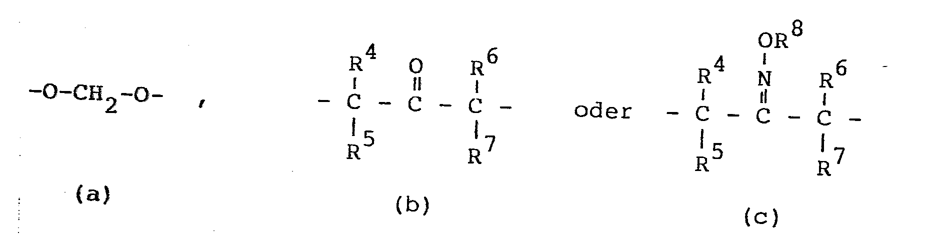 R⁴, R⁵ , R⁶ und R⁷ je niederes Alkyl und R⁸ Wasserstoff oder niederes Alkyl bedeuten,
und deren Säureadditionssalze.

2. Verbindungen gemäss Anspruch 1, worin A einen Rest der in Anspruch 1 definierten Formel (b) oder (c) bedeutet.

3. Verbindungen gemäss Anspruch 2, worin A einen Rest der in Anspruch 1 definierten Formel (b) bedeutet, wobei R⁴, R⁵, Rund R je Methyl bedeuten.

4. Verbindungen gemäss Anspruch 2, worin A einen Rest der in Anspruch 1 definierten Formel (c) bedeutet, wobei R⁴, R⁵, Rund R⁷ je Methyl und R⁸ Wasserstoff oder Methyl bedeuten.

5. Verbindungen gemäss einem der Ansprüche 1 bis 4, worin R² Methyl oder Aethyl bedeutet.

6. Verbindungen gemäss einem der Ansprüche ₁ bis 5, worin R¹ Wasserstoff und R³ Methyl bedeuten.

7. Verbindungen gemäss einem der Ansprüche 1 bis 5, worin _{R}¹ Methyl und R³ Wasserstoff bedeuten.

8. Verbindungen gemäss einem der Ansprüche 1 bis 5, worin _{R}¹ und R³ je Methyl bedeuten.

9. 5,7-Dihydro-2-[[(4-methoxy-3-methyl-2-pyridyl)-methyl]thio]-5,5,7,7-tetramethylindeno[5,6-d]imidazol-6(lH)-on.

10. 5,7-Dihydro-2-[[(4-methoxy-3-methyl-2-pyridyl)-methyl]sulfinyl]-5,5,7,7-tetramethylindeno[5,6-d]imidazol-6(1H)-on.

11. 2-[[(4-Aethoxy-3-methyl-2-pyridyl)methyl]thio]-5,7-dihydro-5,5,7,7-tetramethylindeno[5,6-d]imidazol-6(lH)-on.

12. 2-[[(4-Aethoxy-3-methyl-2-pyridyl)methyl]sulfinyl]-5,7-dihydro-5,5,7,7-tetramethylindeno[5,6-d]imidazol-6(lH)-on.

13. 5,7-Dihydro-2-[[(4-methoxy-3,5-dimethyl-2-pyridyl)-methyl]thio]-5,5,7,7-tetramethylindeno[5,6-d]imidazol-6(lH)-on.

14. 5,7-Dihydro-2-[[(4-methoxy-3,5-dimethyl-2-pyridyl)-methyl]sulfinyl]-5,5,7,7-tetramethylindeno[5,6-d]imidazol-6(lH)-on.

15. 5,7-Dihydro-2-[[(4-methoxy-5-methyl-2-pyridyl) methyl]thio]-5,5,7,7-tetramethylindeno[5,6-d]imidazol-6(lH)-on.

16. 5,7-Dihydro-2-[[(₄-methoxy-5-methyl-2-pyridyl) methyl]sulfinyl]-5,5,7,7-tetramethylindeno[5,6-d]imidazol-6(lH)-on.

17. 2-[[(4-Aethoxy-5-methyl-2-pyridyl)methyl]thiol-5,7-dihydro-5,5,7,7-tetramethylindeno[5,6-d]imidazol-6(1H)-on.

18. 2-[[(4-Aethoxy-5-methyl-2-pyridyl)methyl]sulfinyl]-5,7-dihydro-5,5,7,7-tetramethylindeno[5,6-d]imidazol-6(1H)-on.

19. 5,7-Dihydro-2-[[(4-methoxy-3-methyl-2-pyridyl) methyl]thiol-5,5,7,7-tetramethylindeno[5,6-d]imidazol-6(1H)-on-O-methyloxim.

20. 5,7-Dihydro-2-[[(4-methoxy-3-methyl-2-pyridyl) methyllsulfinyl]-5,5.7,7-tetramethylindeno[5,6-d]imidazol-6(1H)-on-O-methyloxim.

21. 6-[[(4-Methoxy-3-methyl-2-pyridyl)methyl]thio]-5H-1,3-dioxolo[4,5-f]benzimidazol,
6-[[(4-Methoxy-3-methyl-2-pyridyl)methyl]sulfinyl]-5H-1,3-dioxolo[4,5-f]benzimidazol.
6-[[(4-Metboxy-3,5-dimethyl-2-pyridyl)methyl]thio]-5H-1,3-dioxolo[4,5-f]benzimidazol und
6-[[(4-Methoxy-3,5-dimethyl-2-pyridyl)methyl]sulfinyl]-5H-1,3-dioxolo[4,5-f]benzimidazol.

22. Verbindungen der allgemeinen Formel 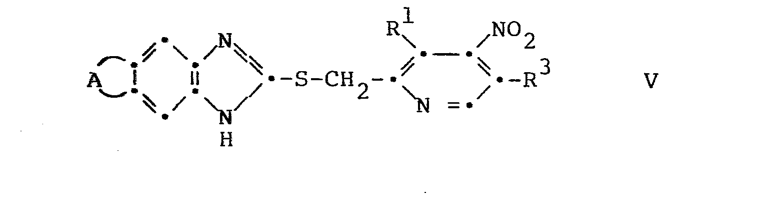 worin _{R}¹, _{R}³ und A die in Anspruch 1 angegebene Bedeutung besitzen.

23. Verbindungen gemäss einem der Ansprüche 1 bis 21 zur Anwendung als therapeutische Wirkstoffe.

24. Verbindungen gemäss einem der Ansprüche 1 bis 21 zur Anwendung als therapeutische Wirkstoffe bei der Bekämpfung bzw. Verhütung von Ulcera und von erhöhter Magensäuresekretion.

25. 5,7-Dihydro-2-[[(4-methoxy-3-methyl -2-pyridyl)-methyl]thio] -5,5,7,7-tetramethylindeno[5,6-d]imidazol--6(lH)-on als Verbindung gemäss Anspruch 23 oder 24.

26. Verfahren zur Herstellung von Verbindungen gemäss einem der Ansprüche 1 bis 21, dadurch gekennzeichnet, dass man
a) eine Verbindung der allgemeinen Formel 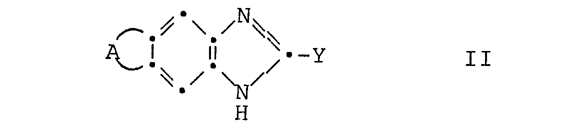 worin A die in Anspruch 1 und Y die unten angegebene Bedeutung besitzen,
mit einer Verbindung der allgemeinen Formel 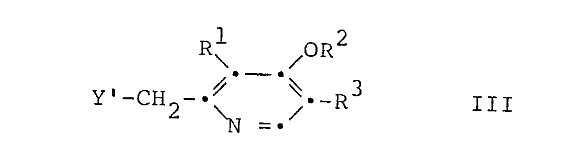 worin _{R}¹, _{R}² und _{R}³ die in Anspruch 1 und Y' die unten angegebene Bedeutung besitzen,
umsetzt, wobei eines von Y und Y' eine Mercaptogruppe und das andere eine Abgangsgruppe bedeutet; oder
b) eine Verbindung der in Anspruch 1 definierten Formel I, worin n die Zahl 0 bedeutet, zu einer entsprechenden Verbindung, worin n die Zahl 1 bedeutet, oxydiert; oder
c) eine Verbindung der in Anspruch 1 definierten Formel I, worin A einen Rest der in Anspruch 1 definierten Formel (b) und n die Zahl 0 bedeuten, mit einer Verbindung der allgemeinen Formel 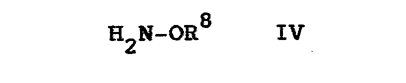 worin R⁸ die in Anspruch 1 angegebene Bedeutung besitzt,
umsetzt; oder
d) eine Verbindung der allgemeinen Formel 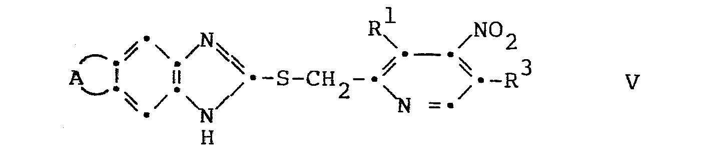 worin R¹, R³ und A die in Anspruch 1 angegebene Bedeutung besitzen,
mit einem eine niedere Alkoxygruppe liefernden Mittel behandelt; oder
e) eine Verbindung der allgemeinen Formel 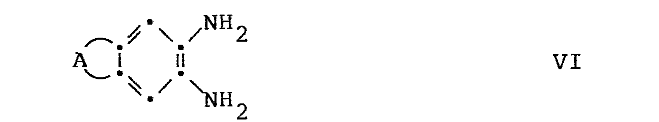 worin A die in Anspruch 1 angegebene Bedeutung besitzt, mit einer Verbindung der allgemeinen Formel 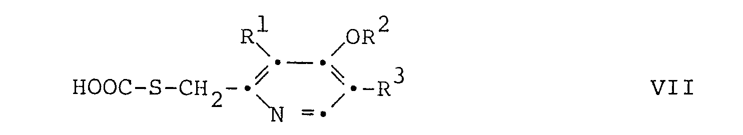 worin R¹, R² und _{R}³ die in Anspruch 1 angegebene Bedeutung besitzen,
umsetzt; oder
f) eine Verbindung der allgemeinen Formel 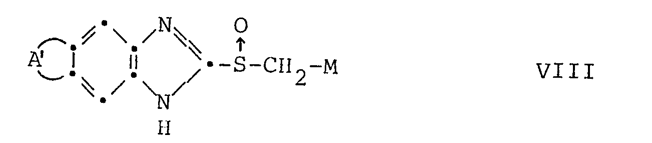 worin A¹ einen Rest der in Anspruch 1 definierten Formel (a), (b) oder (c) bedeutet, wobei aber in Formel (c) R⁸ niederes Alkyl bedeutet, und M ein Alkalimetallatom bedeutet,
mit einer Verbindung der allgemeinen Formel 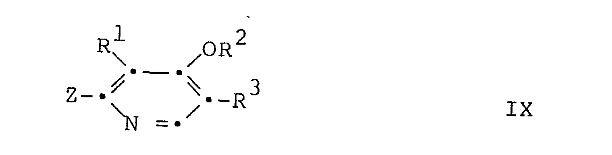
worin _{R}¹, R2 und _{R}³ die in Anspruch 1 angegebene Bedeutung besitzen und Z eine Abgangsgruppe bedeutet, umsetzt;
und erwünschtenfalls eine erhaltene freie Base in ein Säureadditionssalz und/oder ein erhaltenes Säureadditionssalz in die freie Base oder in ein anderes Säureadditionssalz überführt.

27. Verfahren gemäss Anspruch 26, dadurch gekennzeichnet, dass man 5,7-Dihydro-2-[[(4-methoxy-3-methyl -2-pyridyl)methyl]thio] -5,5,7,7-tetramethylindeno[5,6-d]imidazol--6(lH)-on herstellt.

28. Verfahren gemäss Anspruch 26, dadurch gekennzeichnet, dass man Verbindungen der in Anspruch 1 definierten Formel I, worin A einen Rest der in Anspruch 1 definierten Formel (a) bedeutet, gemäss Verfahrensvariante d), e) oder f) herstellt.

29. Arzneimittel, enthaltend eine Verbindung gemäss einem der Ansprüche 1 bis 21 und ein therapeutisch inertes Excipiens.

30. Arzneimittel gemäss Anspruch 29 zur Anwendung bei der Bekämpfung bzw. Verhütung von Ulcera und von erhöhter Magensäuresekretion.

31. Arzneimittel gemäss Anspruch 29 oder 30, enthaltend 5,7-Dihydro-2-[[(4-methoxy-3-methyl -2-pyridyl)methyl]thio] -5,5,7,7-tetramethylindeno[5,6-d]imidazol-6(1H)-on.

32. Verwendung von Verbindungen gemäss einem der Ansprüche 1 bis 21 bei der Bekämpfung bzw. Verhütung von Krankheiten.

3₃. Verwendung von Verbindungen gemäss einem der Ansprüche 1 bis 21 bei der Bekämpfung bzw. Verhütung von Ulcera und von erhöhter Magensäuresekretion.

34. Verwendung von Verbindungen gemäss einem der Ansprüche 1 bis 21 zur Herstellung von Arzneimitteln für die Bekämpfung bzw. Verhütung von Ulcera und von erhöhter Magensäuresekretion.

35. Verwendung von 5,7-Dihydro-2-[[(4-methoxy-3-methyl -2-pyridyl)methyl]thio] -5,5,7,7-tetramethylindeno[5,6-d]-imidazol-6(lH)-on gemäss einem der Ansprüche 32-34.

Patentansprüche für Oesterreich 1. Verfahren zur Herstellung von tricyclischen Imidazolderivaten der allgemeinen Formel 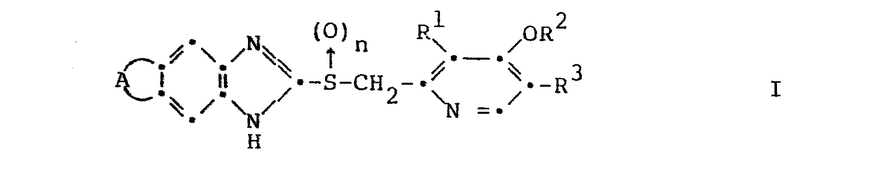 worin eines von R¹ und R³ niederes Alkyl und das andere Wasserstoff oder niederes Alkyl. R² niederes Alkyl, n die Zahl 0 oder 1, A einen Rest der Formel 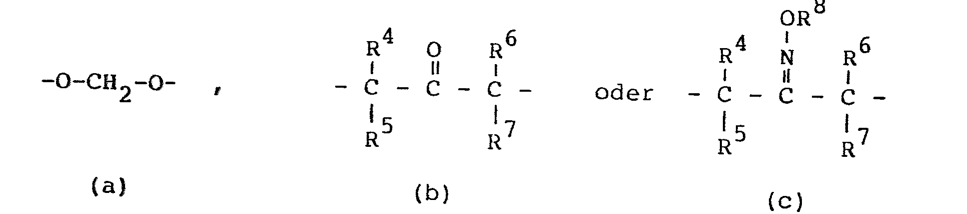 R⁴, R⁵, R⁶ und R⁷ je niederes Alkyl und R⁸ Wasserstoff oder niederes Alkyl bedeuten,
und von deren Säureadditionssalzen, dadurch gekennzeichnet, dass man
a) eine Verbindung der allgemeinen Formel 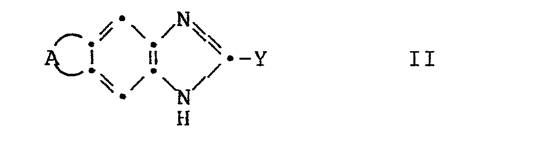 worin A obige und Y die unten angegebene Bedeutung besitzen,
mit einer Verbindung der allgemeinen Formel 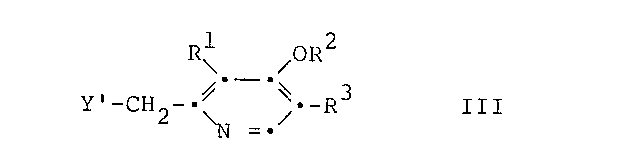 worin R¹, R² und R³ obige und Y' die unten angegebene Bedeutung besitzen,
umsetzt, wobei eines von Y und Y' eine Mercaptogruppe und das andere eine Abgangsgruppe bedeutet; oder
b) eine Verbindung der obigen Formel I, worin n die Zahl 0 bedeutet, zu einer entsprechenden Verbindung, worin n die Zahl 1 bedeutet, oxydiert; oder
c) eine Verbindung der obigen Formel I, worin A einen Rest der obigen Formel (b) und n die Zahl 0 bedeuten, mit einer Verbindung der allgemeinen Formel 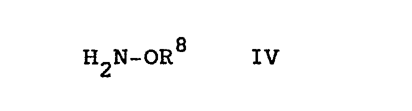 worin R⁸ obige Bedeutung besitzt, umsetzt; oder
d) eine Verbindung der allgemeinen Formel 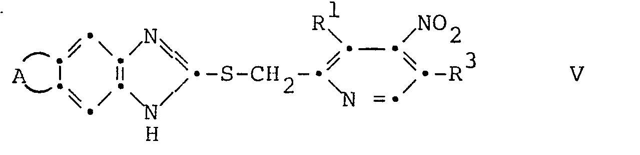 worin R¹, R³ und A obige Bedeutung besitzen,
mit einem eine niedere Alkoxygruppe liefernden Mittel behandelt; oder
e) eine Verbindung der allgemeinen Formel 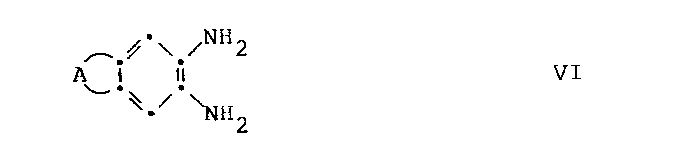 worin A obige Bedeutung besitzt,
mit einer Verbindung der allgemeinen Formel 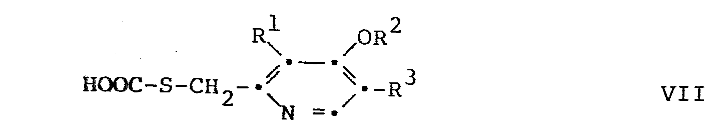 worin R¹, n² und _{R}³ obige Bedeutung besitzen, umsetzt ; oder
f) eine Verbindung der allgemeinen Formel 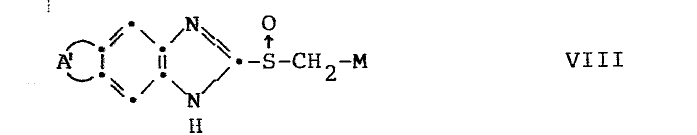 worin A' einen Rest der obigen Formel (a), (b) oder (c) bedeutet, wobei aber in Formel (c) R⁸ niederes Alkyl bedeutet, und M ein Alkalimetallatom bedeutet,
mit einer Verbindung der allgemeinen Formel 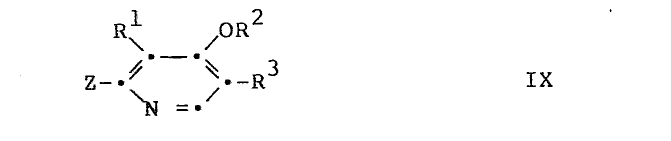 worin R¹, R² und R³ obige Bedeutung besitzen und Z eine Abgangsgruppe bedeutet,
umsetzt ;
und erwünschtenfalls eine erhaltene freie Base in ein Säureadditionssalz und/oder ein erhaltenes Säureadditionssalz in die freie Base oder in ein anderes Säureadditionssalz überführt.

2. Verfahren gemäss Anspruch 1, dadurch gekennzeichnet, dass A einen Rest der in Anspruch 1 definierten Formel (b) oder (c) bedeutet.

3. Verfahren gemäss Anspruch 2, dadurch gekennzeichnet, dass A einen Rest der in Anspruch 1 definierten Formel (b) bedeutet, wobei R⁴, R⁵, R6 und _{R}⁷ je Methyl bedeuten.

4. Verfahren gemäss Anspruch 2, dadurch gekennzeichnet, dass A einen Rest der in Anspruch 1 definierten Formel (c) bedeutet, wobei R⁴, R⁵, Rund R je Methyl und _{R}⁸ Wasserstoff oder Methyl bedeuten.

5. Verfahren gemäss einem der Ansprüche 1 bis 4, dadurch gekennzeichnet, dass R² Methyl oder Aethyl bedeutet.

6. Verfahren gemäss einem der Ansprüche 1 bis 5, dadurch gekennzeichnet, dass R¹ Wasserstoff und _{R}³ Methyl bedeuten.

7. Verfahren gemäss einem der Ansprüche 1 bis 5, dadurch gekennzeichnet, dass R¹ Methyl und R³ Wasserstoff bedeuten.

8. Verfahren gemäss einem der Ansprüche 1 bis 5, dadurch gekennzeichnet, dass R¹ und _{R}³ je Methyl bedeuten.

9. Verfahren gemäss Anspruch 1, dadurch gekennzeichnet, dass man 5,7-Dihydro-2-[[(4-methoxy-3-methyl-2-pyridyl)-methyl]thio]-5,5,7,7-tetramethylindeno[5,6-d]imidazol-6(lH)-on herstellt.

10. Verfahren gemäss Anspruch 1, dadurch gekennzeichnet, dass man 5,7-Dihydro-2-[[(4-methoxy-3-methyl-2-pyridyl)-methyl]sulfinyl]-5,5,7,7-tetramethylindeno[5,6-d]imidazol-6(lH)-on herstellt.

11. Verfahren gemäss Anspruch 1, dadurch gekennzeichnet, dass man 2-[[(4-Aethoxy-3-methyl-2-pyridyl)methyl]-thio]-5,7-dihydro-5,5,7,7-tetramethylindeno[5,6-d]imidazol--6(lH)-on herstellt.

12. Verfahren gemäss Anspruch 1, dadurch gekennzeichnet, dass man 2-[[(4-Aethoxy-3-methyl-2-pyridyl)methyl]-sulfinyl]-5,7-dihydro-5,5.7.7-tetramethylindeno[5,6-d]imidazol-6(lH)-on herstellt.

13. Verfahren gemäss Anspruch 1, dadurch gekennzeichnet, dass man 5,7-Dihydro-2-[[(4-methoxy-3,5-dimethyl-2--pyridyl)-methyl]thio]-5,5,7,7-tetramethylindeno[5,6-d]-imidazol-6(lH)-on herstellt.

14. Verfahren gemäss Anspruch 1, dadurch gekennzeichnet, dass man 5,7-Dihydro-2-[[(4-methoxy-3,5-dimethyl-2--pyridyl)-methyl]sulfinyl]-5,5,7,7-tetramethylindeno-[5,6-d]imidazol-6(lH)-on herstellt.

15. Verfahren gemäss Anspruch 1, dadurch gekennzeichnet, dass man 5,7-Dihydro-2-[[(4-methoxy-5-methyl-2-pyridyl)methyl]thio]-5.5,7,7-tetramethylindeno[5,6-d]imidazol-6(lH)-on herstellt.

16. Verfahren gemäss Anspruch 1, dadurch gekennzeichnet, dass man 5,7-Dihydro-2-[[(4-methoxy-5-methyl-2-pyridyl)methyl]suifinyl]-5,5,7,7-tetramethylindeno[5,6-d]imidazol-6(lH)-on herstellt.

17. Verfahren gemäss Anspruch 1, dadurch gekennzeichnet, dass man 2-[[(4-Aethoxy-5-methyl-2-pyridyl)methyl]-thio]-5,7-dihydro-5,5,7,7-tetramethylindeno[5,6-d]imidazol-6(lH)-on herstellt.

18. Verfahren gemäss Anspruch 1, dadurch gekennzeichnet, dass man 2-[[(4-Aethoxy-5-methyl-2-pyridyl)methyl]-sulfinyl]-5,7-dihydro-5,5,7,7-tetramethylindeno[5,6-d]imidazol-6(lH)-on herstellt.

19. Verfahren gemäss Anspruch 1, dadurch gekennzeichnet, dass man 5,7-Dihydro-2-[[(4-methoxy-3-methyl-2-pyridyl)methyl]thio]-5,5,7,7-tetramethylindeno[5,6-d]imidazol--6(lH)-on-O-methyloxim herstellt.

20. Verfahren gemäss Anspruch 1, dadurch gekennzeichnet, dass man 5,7-Dihydro-2-[[(4-methoxy-3-methyl-2-pyridyl)methyl]sulfinyl]-5,5,7,7-tetramethylindeno[5,6-d]imidazol-6(lH)-on-O-methyloxim herstellt.

21. Verfahren gemäss Anspruch 1, dadurch gekennzeichnet, dass man Verbindungen der in Anspruch 1 defininerten Formel I, worin A einen Rest der in Anspruch l definierten Formel (a) bedeutet, gemäss Verfahrensvariante d), e) oder f) herstellt.
